# EUROPEAN PATENT APPLICATION

(11) **EP 3 184 674 A1**
(43) Date of publication of application: **28.06.2017**
(21) Application number: 15202448.5
(22) Date of filing: 23.12.2015
(51) Int. Cl.: C40B 40/06, C07H 21/00

(54) **DNA-ENCODED CHEMICAL LIBRARY, USE THEREOF AND METHOD TO SYNTHESIZE THE LIBRARY**

(71) Applicant: Technische Universität Dortmund, 44221 Dortmund (DE)
(72) Inventor: Brunschweiger, Andreas, 44265 Dortmund (DE); Krause, Norbert, 44287 Dortmund (DE); Antonchick, Andrey, 44227 Dortmund (DE); Klika Skopic, Mateja, 44388 Dortmund (DE); Salamon, Hazem, 44225 Dortmund (DE); Bugain, Olivia, 44388 Dortmund (DE); Jung, Kathrin, 47137 Duisburg (DE); Wagner, Bernd, 44137 Dortmund (DE)
(74) Representative: Viering, Jentschura & Partner mbB Patent- und Rechtsanwälte

(57) **Abstract**

The present invention relates to a compound library comprising a plurality of conjugate molecules, wherein said conjugates comprise a small organic molecule covalently coupled to a nucleic acid moiety, wherein the nucleic acid moiety consists of pyrimidine nucleotides and the plurality of conjugates differ from each other at least by comprising different organic molecules. Further, the present invention relates to the use of said library for screening compounds binding to a target molecule and methods of synthesizing said library.

SEQUENCE LISTING

<110>  TU Dortmund

<120>  DNA-ENCODED CHEMICAL LIBRARY, USE THEREOF AND METHOD TO
       SYNTHESIZE THE LIBRARY

<130>  P62368

<160>  29

<170>  PatentIn version 3.5

<210>  1
<211>  6
<212>  DNA
<213>  Artificial Sequence

<220>
<223>  DNA involved in ligation

<400>  1
tttttt                                                                  6


<210>  2
<211>  31
<212>  DNA
<213>  Artificial Sequence

<220>
<223>  DNA involved in ligation

<400>  2
gtcttgccga attccgctgt cataacatag g                                     31


<210>  3
<211>  41
<212>  DNA
<213>  Artificial Sequence

<220>
<223>  DNA involved in ligation

<400>  3
taggcctatg ttatgacagc ggaattcggc aagacaaaaa a                          41


<210>  4
<211>  37
<212>  DNA
<213>  Artificial Sequence

<220>
<223>  DNA involved in ligation

<400>  4
ttttttgtct tgccgaattc cgctgtcata acatagg                                37


<210>  5
<211>  30

<212>  DNA
<213>  Artificial Sequence

<220>
<223>  DNA involved in ligation

<400>  5
cctaactgtt cacgactgct gtgtgacttc                                        30


<210>  6
<211>  26
<212>  DNA
<213>  Artificial Sequence

<220>
<223>  DNA involved in ligation

<400>  6
gaagtcacac agcagtcgtg aacagt                                            26


<210>  7
<211>  67
<212>  DNA
<213>  Artificial Sequence

<220>
<223>  DNA involved in ligation

<400>  7
ttttttgtct tgccgaattc cgctgtcata acataggcct aactgttcac gactgctgtg        60

tgacttc                                                                 67


<210>  8
<211>  67
<212>  DNA
<213>  Artificial Sequence

<220>
<223>  DNA involved in ligation

<400>  8
gaagtcacac agcagtcgtg aacagttagg cctatgttat gacagcggaa ttcggcaaga       60

caaaaaa                                                                 67


<210>  9
<211>  15
<212>  DNA
<213>  Artificial Sequence

<220>
<223>  DNA involved in ligation

<400>  9
tttttgtct tgccg                                                         15


<210>  10

<211>  22
<212>  DNA
<213>  Artificial Sequence

<220>
<223>  DNA involved in ligation

<400>  10
aattccgctg tcataacata gg                                                22


<210>  11
<211>  19
<212>  DNA
<213>  Artificial Sequence

<220>
<223>  DNA involved in ligation

<400>  11
aattcggcaa gacaaaaaa                                                    19


<210>  12
<211>  22
<212>  DNA
<213>  Artificial Sequence

<220>
<223>  DNA involved in ligation

<400>  12
taggcctatg ttatgacagc gg                                                22


<210>  13
<211>  6
<212>  DNA
<213>  Artificial Sequence

<220>
<223>  DNA involved in ligation

<400>  13
aaaaaa                                                                   6


<210>  14
<211>  18
<212>  DNA
<213>  Artificial Sequence

<220>
<223>  DNA involved in ligation

<400>  14
gtcttgccga attccgct                                                     18


<210>  15
<211>  18
<212>  DNA
<213>  Artificial Sequence

<220>
<223>  DNA involved in ligation

<400>  15
agcggaattc ggcaagac                                                    18


<210>  16
<211>  5
<212>  DNA
<213>  Artificial Sequence

<220>
<223>  DNA involved in ligation

<400>  16
gtcat                                                                   5


<210>  17
<211>  5
<212>  DNA
<213>  Artificial Sequence

<220>
<223>  DNA involved in ligation

<400>  17
atgac                                                                   5


<210>  18
<211>  8
<212>  DNA
<213>  Artificial Sequence

<220>
<223>  DNA involved in ligation

<400>  18
aacatagg                                                                8


<210>  19
<211>  8
<212>  DNA
<213>  Artificial Sequence

<220>
<223>  DNA involved in ligation

<400>  19
cctatgtt                                                                8


<210>  20
<211>  4
<212>  DNA
<213>  Artificial Sequence

<220>
<223>  DNA involved in ligation

<400>  20
ccta                                                                    4


<210>  21
<211>  4
<212>  DNA
<213>  Artificial Sequence

<220>
<223>  DNA involved in ligation

<400>  21
tagg                                                                    4


<210>  22
<211>  8
<212>  DNA
<213>  Artificial Sequence

<220>
<223>  DNA involved in ligation

<400>  22
actgttca                                                                8


<210>  23
<211>  8
<212>  DNA
<213>  Artificial Sequence

<220>
<223>  DNA involved in ligation

<400>  23
tgaacagt                                                                8


<210>  24
<211>  18
<212>  DNA
<213>  Artificial Sequence

<220>
<223>  DNA involved in ligation

<400>  24
cgactgctgt gtgacttc                                                    18


<210>  25
<211>  18
<212>  DNA
<213>  Artificial Sequence

<220>
<223>  DNA involved in ligation

<400>  25
gaagtcacac agcagtcg                                                    18

<210>  26
<211>  9
<212>  DNA
<213>  Artificial Sequence

<220>
<223>  DNA involved in ligation

<400>  26
gtcttgccg                                                                9


<210>  27
<211>  9
<212>  DNA
<213>  Artificial Sequence

<220>
<223>  DNA involved in ligation

<400>  27
aattccgct                                                                9


<210>  28
<211>  13
<212>  DNA
<213>  Artificial Sequence

<220>
<223>  DNA involved in ligation

<400>  28
aattcggcaa gac                                                          13


<210>  29
<211>  5
<212>  DNA
<213>  Artificial Sequence

<220>
<223>  DNA involved in ligation

<400>  29
agcgg                                                                    5

## Description

### FIELD OF THE INVENTION

The present invention lies in the field of medicinal chemistry and chemical biology and relates to a compound library comprising a plurality of conjugate molecules, wherein said conjugates comprise a small organic molecule covalently coupled to a nucleic acid moiety, wherein the nucleic acid moiety consists of pyrimidine nucleotides and the plurality of conjugates differ from each other at least by comprising different organic molecules. Further, the present invention relates to the use of said library for screening compounds binding to a target molecule and methods of synthesizing said library.

### BACKGROUND OF THE INVENTION

DNA-encoded chemical libraries (DECLs) represent a tool for drug discovery. DECL technology allows the synthesis and screening of chemical libraries of unprecedented size at moderate costs. DECLs feature the display of individual small organic chemical moieties on DNA fragments serving as amplifiable identification barcodes. The DNA-tag allows the simultaneous screening of a very large set of compounds (up to billions of molecules), because the hit compounds can easily be identified and quantified by PCR-amplification and sequencing of the DNA-barcode. Several approaches have been used to generate DECLs. Most common is the combinatorial mix-and-split synthesis strategy in which preparative organic synthesis and encoding steps are performed in iterative manner.

A prerequisite for the library synthesis is the compatibility of DNA with the synthesis methodology used for synthesis of the organic chemical moieties. Currently, reaction methodology meeting this requirement is very limited. Therefore, DECLs are biased towards a certain chemical space, contradicting the endeavor to design screening libraries to cover chemical space as broad as possible. A current challenge for DECL synthesis research is the development of novel synthetic schemes that furnish in DNA-compatible manner DNA-conjugates of small and geometrically defined (rigid) scaffolds which serve as starting points for subsequent combinatorial library synthesis.

The synthesis of such molecules would result in molecules having a drug-like structure. These drug-like structures include, but are not limited to heterocyclic structures.

Surprisingly, the present inventors found a synthesis strategy that enables the synthesis of several (hetero)cyclic structures from different starting materials attached to a specific DNA sequence by various catalytic methods. Fundamental to the present invention is the observation that DNA-sequences that contain only pyrimidine nucleotides are tolerant to a broad spectrum of reaction conditions, such as the use of strong acids or transition metals as catalysts, while, as commonly known in the field, DNA-molecules comprising purines are degraded under the same reaction conditions by depurination and subsequent strand fragmentation of the resulting abasic sites. The possibility to use a broad spectrum of reaction conditions allows the synthesis of several heterocyclic structures attached to DNA-sequences. In turn, this possibility to apply a broader spectrum of chemical reaction conditions results in the synthesis of molecules that have a drug-like structure.

### SUMMARY OF THE INVENTION

In a first aspect, the present invention relates to a compound library comprising a plurality of conjugate molecules, wherein said conjugates comprise a small organic molecule covalently coupled to a nucleic acid moiety, wherein the nucleic acid moiety consists of pyrimidine nucleotides and the plurality of conjugates differ from each other at least by comprising different organic molecules.

In various embodiments, the plurality of conjugate molecules comprises at least ten molecules, preferably at least 50 different molecules, more preferably at least 100 different molecules.

In various other embodiments, the nucleic acid moiety consists of two to 30, preferably three to ten, more preferably six nucleotides.

In still various other embodiments, the nucleic acid moiety is RNA, DNA or a mixture thereof. In more preferred embodiments, the nucleic acid portion is DNA.

In various embodiments, the nucleic acid moiety and the organic molecule are linked by amide bond.

In further embodiments, the present invention relates to a compound library wherein the conjugates differ from each other by comprising different nucleic acid moieties.

In various embodiments, the conjugate molecules of the library further comprise a linker portion between the nucleic acid moiety and the organic moiety molecule, preferably this linker portion is polyethylene glycol (PEG).

In various other embodiments, the organic molecule consists of 2 or more carbon atoms, preferably up to 50 carbon atoms, more preferably up to 30 carbon atoms. In other various embodiments, the organic molecule has a molecular weight of at most 900 daltons, preferably at most 700 daltons and more preferably at most 500 daltons.

In still various other embodiments, the pyrimidine nucleotides are selected from the group consisting of thymidine, cytidine, uridine, 4-acetylcytidine, 5-(carboxyhydroxymethyl)uridine, 2'-O-methylcytidine, 5-carboxymethylaminomethyl-2-thiouridine, 5-carboxymethylaminomethyluridine, dihydrouridine, 2'-O-methylpseudouridine, 1-methylpseudouridine, 3-methylcytidine, 5-methylcytidine, 5-methylaminomethyluridine, 5-methoxyaminomethyl-2-thiouridine, 5-methoxycarbonylmethyl-2-thiouridine, 5-methoxycarbonylmethyluridine, 5-methoxyuridine, uridine-5-oxyacetic acid-methylester, uridine-5-oxyacetic acid, pseudouridine, 2-thiocytidine, 5-methyl-2-thiouridine, 2-thiouridine, 4-thiouridine, 5-methyluridine, 2'-O-alkyluridine, 2'-O-alkylthymidine, 2'-O-alkylcytidine and 3-(3-amino-3-carboxy-propyl)uridine.

In a further aspect, the present invention relates to a method for synthesizing a compound library, wherein said library comprises a plurality of conjugate molecules, wherein said conjugates comprise a small organic molecule covalently coupled to a nucleic acid moiety, the synthesis of each conjugate molecule comprises: reacting a nucleic acid consisting of pyrimidine nucleotides with an organic molecule under conditions that allow the conjugation of said molecules, wherein the plurality of conjugate molecules differ from each other at least by comprising different organic molecules.

In various embodiments of the method, the conjugate molecule is further used in a reaction comprising a) a strong acid, preferably an acid having a pKₐ of less than 3; and/or b) a catalyst selected from the group consisting of copper, silver, gold, ruthenium, iron, osmium, cobalt, rhodium, iridium, nickel, palladium, platinum and derivatives thereof, preferably copper, silver, gold, ruthenium and derivatives thereof.

In still various embodiments of the method, the nucleic acid moiety is elongated in an additional reaction step with pyrimidine and/or purine nucleotides after the conjugation step.

In a third aspect, the present invention relates to the use of a compound library of the present invention for screening a compound binding to a target molecule.

In various embodiments, the target molecule is a protein.

It is understood that all combinations of the above disclosed embodiments are also intended to fall within the scope of the present invention.

### BRIEF DESCRIPTION OF THE DRAWINGS

The invention will be better understood with reference to the detailed description when considered in conjunction with the non-limiting examples and the accompanying drawings.
**Figure 1** shows the synthesis of DNA-amide conjugates.
**Figure 2** shows the removal of the Fmoc-protective group from DNA-amide conjugates.
**Figure 3** shows the synthesis of the 3'-5'-hexathymidine-5'-tryptophane conjugate **2**.
**Figure 4** shows the synthesis of the 3'-5'-hexathymidine-5'-β-carboline conjugates **3a-3p.**
Figure 5 (a) shows the HPLC chromatogram of compound **2**; Figure 5 (b) shows the HPLC chromatogram of compound **4a**; Figure 5 (c) shows the MALDI-MS analysis of compound **4a**.
**Figure 6** shows the ring-closing metathesis of a 3'-5'-hexathymidine-5'-peptide conjugate catalyzed by the ruthenium catalyst bis(tricyclohexylphosphine)benzylidine ruthenium(IV) dichloride yielding **6a**.
**Figure 7** (**a**) shows the HPLC chromatogram of compound **5**; Figure 8 (b) shows the HPLC chromatogram of compound **6a**; Figure 8 (c) shows the MALDI-MS analysis of compound **6a**.
**Figure 8** shows the synthesis of the 3'-5'-hexathymidine-5'-PEG-linker conjugate **9**.
**Figure 9** shows the synthesis of the 3'-5'-hexathymidine-5'-aldehyde conjugate **11**.
**Figure 10** shows the gold-catalyzed synthesis of the 3'-5'-hexathymidine-5'-6-oxa-1,2-diazaspiro[4.4]nonane-conjugate **14**.
**Figure 11** (**a**) shows the HPLC chromatogram of the 3'-5'-hexathymidine-5'-conjugate **11**; **Figure 13** (**b**) shows the HPLC chromatogram of DNA-conjugate **14**; Figure 13 (c) shows the MALDI-TOF analysis of the 3'-5'-hexathymidine-5'-conjugate **14**, expected mass= 2640,14; found mass= 2639,45.
**Figure 12** shows the synthesis of the 3'-5'-hexathymidine-5'-alkyne conjugate **16**.
**Figure 13** shows the gold-catalyzed synthesis of 3'-5'-hexathymidine-5'-pyrazoline-conjugate **19**.
Figure 14 (a) shows the HPLC chromatogram of the 3'-5'-hexathymidine-5'-conjugate **16**; Figure 14 (b) shows the HPLC chromatogram of DNA-conjugate **19**; Figure 14 (c) shows the MALDI-TOF analysis of hexathymidine-5'-conjugate **19**, expected mass= 239,71; found mass= 2392,256.
**Figure 15** shows the synthesis of *tert*-butyl 4-(3-benzylidene-2-oxoindolin-1-yl)butanoate **25**
**Figure 16** shows the synthesis of the 3'-5'-hexathymidine-5'-oxindole conjugate **27**.
**Figure 17** shows the Ag (I) or Cu(I)-catalyzed synthesis of the 3'-5'-hexathymidine-5'-spirooxindole conjugate **29a**.
Figure 18 (a) shows the HPLC chromatogram of compound **27**; Figure 20 (b) shows the HPLC chromatogram of compound **29a**; Figure 20 (c) shows the MALDI-TOF analysis of the hexathymidine-5'-conjugate **29a**, expected mass= 2733,09; found mass= 2733,014.
**Figure 19** shows the first ligation reaction, ligating a coding DNA to the "adapter nucleic acid".
**Figure 20** shows the second ligation reaction.
**Figure 21** shows the agarose gel electrophoresis of ligations 1 and 2 having the following arrangement: **L**: ladder, ThermoScientific GeneRuler Ultra Low Range DNA Ladder; **lane 1**: oligonucleotides **I, II** (phosphorylated), and **III; lane 2:** T4 DNA ligase reaction yielding a duplex of oligonucleotides **III, IV;** lane 3: oligonucleotides **III** (phosphorylated), **IV, V** (phosphorylated), and **VI; lane 4:** T4 DNA ligase reaction yielding a duplex of oligonucleotides **VII, VIII** 5,5%ig agarose gel; 75minutes; 150V; 25pmol per lane;gel stain: MIDORIGREEN DIRECT (Biozym).
**Figure 22** shows an EcoRI digest and result of MALDI-TOF analysis.

### DETAILED DESCRIPTION OF THE INVENTION

The present inventors surprisingly found that only purine nucleotides, but not pyrimidine nucleotides, are degraded under several conditions used for the synthesis of heterocyclic chemical compounds (for example reactions involving the use of strong acids or transition metals such as Au(I), Ag(I), Ru(II), and Cu(I) as catalysts). Based on these results, the present inventors concluded that chemical compounds being labeled with DNA can be reacted to a broad spectrum of chemical structures, including but not limited to heterocycles, if the DNA-label only consists of pyrimidine nucleotides. At a later stage, after the organic moiety of the compound has been reacted to its final chemical structure, it is also possible to elongate the DNA-label with further nucleotides including purine nucleotides.

Thus, in a first aspect, the present invention relates to a compound library comprising a plurality of conjugate molecules, wherein said conjugates comprise a small organic molecule covalently coupled to a nucleic acid moiety, wherein the nucleic acid moiety consists of pyrimidine nucleotides and the plurality of conjugates differ from each other at least by comprising different organic molecules.

The term "compound", as used herein, relates to a substance formed when two or more chemical elements are chemically bonded together. A typical type of chemical bond in the compounds of the present invention is a covalent bond. However, atoms within the compound may also be bound to each other by ionic bonds, metallic bonds and/or coordinate covalent bonds. "Compounds" refer generally to molecules potentially capable of structural interactions with cellular constituents through noncovalent interactions, such as, for example, through hydrogen bonds, ionic bonds, van der Waals attractions, or hydrophobic interactions. For example, compounds will most typically include molecules with moieties necessary for structural interaction with proteins, glycoproteins, and/or other macromolecules. "Compound library", as used herein, refers to any collection of agents/compounds that includes a plurality of molecular structures. Compound libraries can include, for example, combinatorial chemical libraries, natural products libraries, and peptide and cyclized peptide libraries. Compound libraries of the present invention contain compounds that comprise a small organic molecule covalently coupled to a nucleic acid moiety. Such libraries are known as DNA-encoded chemical libraries (DECLs).

"Plurality", as used herein, is defined as two or more than two, in particular 2, 3, 4, 5, 6, 7, 8, 9, 10 or more, more preferably 100 or more, 500 or more, 1000 or more, 1500 or more, 3000 or more, 5000 or more, 10000 or more or 50000 or more. In various embodiments, the library of the present invention comprises at least ten molecules, preferably at least 50 different molecules, more preferably at least 100 different molecules.

The term "conjugate molecule", as used herein, refers to a compound comprising two or more molecules (e.g., proteins, carbohydrates, organic molecules or nucleic acid molecules) that are chemically linked. The two or more molecules desirably are chemically linked using any suitable chemical bond (e.g., covalent bond). Suitable chemical bonds are well known in the art and include amide bonds, disulfide bonds, thioester bonds, and ester bonds. In various embodiments, the compounds comprise a small organic molecule covalently coupled to a nucleic acid moiety. Preferably, the nucleic acid moiety and the organic molecule are linked by amide bond. The term "organic molecule" refers to carbon-based chemicals, including small organic molecules and macromolecules, as well as derivatives and analogues thereof. The organic molecules may display biological activity such as, but not limited to pharmaceutical, antibiotic, pesticidical, herbicidical, or fungicidical activity. Preferably, the organic molecule contains a heterocyclic structure. The term "heterocyclic," as used herein, means an aromatic or non-aromatic saturated mono-, bi-, or tricyclic ring system having 2 to 14 ring carbon atoms, containing 1-5 ring atoms chosen from NH, N-(CO)-C₁₋₆-alkyl, NC₁₋₆-alkyl, O, SO₂, S, Br, Cl, P, or PO₄⁻³ alone or in combination. Bi- and tricyclic heterocyclic groups are fused at 2 or 4 points or joined at one point via a bond or a heteroatom linker (O, S, NH, or N(C₁-C₆ alkyl). The mono-, bi-, or tricyclic heterocyclic can be optionally substituted on the ring by replacing an available hydrogen on the ring by one or more substituents which may be the same or different, each being independently selected from the groups defined below. There are no adjacent oxygen and/or sulfur atoms present in the ring system. The nitrogen or sulfur atom of the heterocyclic ring system can be optionally oxidized to the corresponding N-oxide, S-oxide or S-dioxide. Non-limiting examples of suitable heterocyclic ring system include furanyl, imidazolyl, isoxazolyl, oxadiazolyl, oxazolyl, pyrazolyl, pyrrolyl, pyridyl, pyrimidyl, pyridazinyl, thiazolyl, triazolyl, tetrazolyl, thienyl, carbazolyl, benzimidazolyl, benzothienyl, benzofuranyl, indolyl, quinolinyl, benzotriazolyl, benzothiazolyl, benzooxazolyl, benzimidazolyl, isoquinolinyl, isoindolyl, acridinyl, or benzoisoxazolyl. Non-limiting examples of suitable heterocyclic rings include also aziridinyl, piperidinyl, pyrrolidinyl, piperazinyl, tetrahydropyranyl, tetrahydrofuranyl, tetrahydrothiophenyl, morpholinyl, thiomorpholinyl and the like. Also included within the scope of the term "heterocyclic" as it is used herein is a group in which the heterocyclic ring is fused at two points or joined at one point via a bond or a heteroatom linker (O, S, NH, or N(C₁-C₆ alkyl), to one aromatic, or cycloalkyl ring, non-limiting examples include isoindoline-1,3-dione 1-methyl-2-phenyl-1H-pyrazole-3(2H)-one, indoline, pyridoindole and the like.

The "organic molecule", as used herein, may also refer to molecules of different classes such as small molecules conforming or not conforming to Lipinski's rule of five, (cyclic) peptides, proteins, nucleotides, lipids, sugars and derivatives thereof. In one embodiment, the organic molecule of the present invention can be selected from the group comprising small molecules conforming or not conforming to Lipinski's rule of five, (cyclic) peptides, proteins, nucleotides and mixtures thereof.

"Small" in the context of the term "organic molecule", as used herein, relates to compounds that consist of 2 or more carbon atoms, preferably up to 50 carbon atoms, more preferably up to 30 carbon atoms. In other various embodiments, the organic molecule has a molecular weight of at most 1500daltons, preferably at most 700 daltons and more preferably at most 500 daltons.

The term "nucleic acid moiety", as used herein, refers to a nucleic acid sequence that is covalently coupled to an organic moiety, as defined above, to form a conjugate molecule. The terms "nucleotide", "nucleic acid molecule" or "nucleic acid sequence", as interchangeably used herein, relate to DNA (deoxyribonucleic acid) molecules, RNA (ribonucleic acid) molecules or molecules comprising both, DNA and RNA. Said molecules may appear independent of their natural genetic context and/or background. The term "nucleic acid molecule/sequence" further refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms.

"RNA" or "ribonucleic acid", as interchangeably used herein, relates to a chain of nucleotides wherein the nucleotides contain the sugar ribose and bases selected from the group of adenine (A), cytosine (C), guanine (G), or uracil (U). "DNA" or "deoxyribonucleic acid", as interchangeably used herein, relates to a chain of nucleotides wherein the nucleotides contain the sugar 2'-deoxyribose and bases selected from adenine (A), guanine (G), cytosine (C) and thymine (T). The term "mRNA" refers to messenger RNA.

The term "base" or "nucleobase", as interchangeably used herein, relates to nitrogen-containing biological compounds (nitrogenous bases) found linked to a sugar within nucleosides - the basic building blocks of deoxyribonucleic acid (DNA) and ribonucleic acid (RNA). Their ability to form base pairs and to stack upon one another lead directly to the helical structure of DNA and RNA. The primary, or canonical, nucleobases are cytosine (DNA and RNA), guanine (DNA and RNA), adenine (DNA and RNA), thymine (DNA) and uracil (RNA), abbreviated as C, G, A, T, and U, respectively. Because A, G, C, and T appear in the DNA, these molecules are called DNA-bases; A, G, C, and U are called RNA-bases. Uracil and thymine are identical except that uracil lacks the 5' methyl group. Adenine and guanine belong to the double-ringed class of molecules called purines (abbreviated as R). Cytosine, thymine, and uracil are all pyrimidines (abbreviated as Y). Other bases that do not function as normal parts of the genetic code are termed non-canonical. Nucleobases that can be used to be coupled to the organic molecule to form a compound of the library of the present invention are thymine, cytosine, uracil, 4-acetylcytidine, 5-(carboxyhydroxymethyl)uridine, 2'-O-methylcytidine, 5-carboxymethylaminomethyl-2-thiouridine, 5-carboxymethylaminomethyluridine, dihydrouridine, 2'-O-methylpseudouridine, 1-methylpseudouridine, 3-methylcytidine, 5-methylcytidine, 5-methylaminomethyluridine, 5-methoxyaminomethyl-2-thiouridine, 5-methoxycarbonylmethyl-2-thiouridine, 5-methoxycarbonylmethyluridine, 5-methoxyuridine, uridine-5-oxyacetic acid-methylester, uridine-5-oxyacetic acid, pseudouridine, 2-thiocytidine, 5-methyl-2-thiouridine, 2-thiouridine, 4-thiouridine, 5-methyluridine, 2'-O-alkyluridine, 2'-O-alkylthymidine, 2'-O-alkylcytidine and 3-(3-amino-3-carboxy-propyl)uridine.

The term "alkyl", as used in the context of 2'-O-alkyluridine, 2'-O-alkylthymidine, 2'-O-alkylcytidine, refers to a saturated or unsaturated hydrocarbon containing 1-20 carbon atoms including both acyclic and cyclic structures (such as methyl, ethyl, propyl, isopropyl, butyl, isobutyl, sec-butyl, pentyl, hexyl, cyclopropyl, cyclobutyl, cyclopentyl, cyclohexyl, propenyl, butenyl).

In various other embodiments, the nucleic acid moiety consists of two to 20, preferably three to ten, more preferably six nucleotides.

In still various other embodiments, the nucleic acid moiety is RNA, DNA or a mixture thereof. In more preferred embodiments, the nucleic acid portion is DNA.

In further embodiments, the present invention relates to a compound library wherein the conjugates differ from each other by comprising different nucleic acid moieties. Basically, there are two alternative methods to ensure that each molecule of the plurality of conjugate molecules comprises a nucleic acid moiety that is distinct from the nucleic acid moieties of the other conjugate molecules: first, all individual types of organic moieties are coupled to individual sequences of pyrimidine nucleotides. By this method, the different conjugate molecules can be discriminated from each other directly after the coupling to the individual nucleic acid sequences. In a second method that allows the discrimination of the different conjugate molecules, the different types of organic molecules are linked to one type of nucleic acid sequence that comprises pyrimidine nucleotides, for example six thymine nucleotides. This pyrimidine nucleic acid moiety (of six thymine nucleotides) is a so-called "adaptor nucleic acid" which is elongated with further nucleotides after the synthesis steps have been applied on the organic molecule. The nucleotides used in this elongation step of the adaptor nucleic acid are not limited to pyrimidine nucleotides and can comprise purine nucleotides. The elongation step is used to individualize the nucleic acid moiety sequence attached to each individual organic molecule. A preferred method for the elongation of the nucleic acid moiety is DNA ligation with T4 ligase or with a chemical condensing agent using nucleotides that comprises a nucleic acid sequence that hybridizes with the adaptor nucleic acid and that additionally comprises a nucleic acid sequence which encodes for a sequence that allows the individualization of the conjugate molecule.

"Covalent" or "covalent bond", as used herein, refers to a chemical bond that involves the sharing of electron pairs between atoms. These electron pairs are known as shared pairs or bonding pairs and the stable balance of attractive and repulsive forces between atoms when they share electrons is known as covalent bonding. For many molecules, the sharing of electrons allows each atom to attain the equivalent of a full outer shell, corresponding to a stable electronic configuration. Covalent bonding includes many kinds of interactions, including σ-bonding, π-bonding, metal-to-metal bonding, agostic interactions, bent bonds, and three-center two-electron bonds.

In various embodiments, the conjugate molecules of the library further comprise a linker portion between the nucleic acid moiety and the organic moiety molecule, preferably this linker portion is polyethylene glycol (PEG). Chemical linkers may also be incorporated into the assembly reactants. The chemical linkers may be included between any of the moieties. Chemical linkers may optionally connect two or more of the moieties to introduce additional functionality or facilitate synthesis. The chemical linker can be a bond between any of the moieties. The bond can include a physical interaction, such as chemical bonds (either directly linked or through intermediate structures), or a non-physical interaction or attractive force, such as electrostatic attraction, hydrogen bonding, and van der Waals/dispersion forces. The linker may be conjugated to any given nucleotide within the nucleic acid moiety. In addition, any position of the ribose or the nucleobase within a given nucleotide of the nucleic acid moiety may be used to the coupling of the linker. In preferred embodiments, the linker is coupled to the nucleic acid moiety by the free 5'-position, namely a phosphate group, of the most 5'-terminal nucleotide of the nucleic acid moiety.

The chemical linkers may aid in facilitating spatial separation of the moieties, increasing flexibility of the moieties relative to each other, introducing a cleavage site or modification site to the templated assembly reactant, facilitating synthesis of the templated assembly reactant, improving physical or functional characteristics (such as solubility, hydrophobicity, charge, cell-permeability, toxicity, biodistribution, or stability) of a templated assembly reactant, or any combination of the above. In various embodiments, the chemical linker is derived from a cross-linker that facilitates connecting the assembly reactant moieties via bioconjugation chemistry. "Bioconjugation chemistry", as used herein, refers to the chemical synthesis strategies and reagents that ligate common functional groups together under mild conditions, facilitating the modular construction of multi-moiety compounds. Due to the mild reaction conditions, bioconjugate chemistry approaches can be suitable for ligating biomolecules, such as nucleic acids, peptides, or polysaccharides. Some non-limiting examples can include chains of one or more of the following: alkyl groups, alkenyl groups, amides, esters, thioesters, ketones, ethers, thioethers, disulfides, ethylene glycol, cycloalkyl groups, benzyl groups, heterocyclic groups, maleimidyl groups, hydrazones, urethanes, azoles, imines, haloalkyl groups, carbamates, or combinations of any of these. In preferred embodiments, the linker is polyethylene glycol (PEG).

In addition to chemical linkers between moieties, additional functionality may optionally be introduced to the conjugate molecule by the addition of accessory groups to the moieties. Some non-limiting examples of accessory groups can include appending a chemical tag or fluorophore to track the location of the conjugation molecule, or appending an agent that improves delivery of the conjugate molecule to a given target, stabilizing groups or groups that improve purification of the conjugate molecule.

"At least one", as used herein, relates to one or more, in particular 1, 2, 3, 4, 5, 6, 7, 8, 9, 10 or more.

In a further aspect, the present invention relates to a method for synthesizing a compound library, wherein said library comprises a plurality of conjugate molecules, wherein said conjugates comprise a small organic molecule covalently coupled to a nucleic acid moiety, the synthesis of each conjugate molecule comprises: reacting a nucleic acid consisting of pyrimidine nucleotides with an organic molecule under conditions that allow the conjugation of said molecules, wherein the plurality of conjugate molecules differ from each other at least by comprising different organic molecules.

The term "synthesizing" or "synthesis", as used herein, refers to a purposeful execution of chemical reactions to obtain a product, or several products. This happens by physical and chemical manipulations usually involving one or more reactions. This may imply that the process is reproducible, reliable, and established to work in multiple laboratories.

A chemical synthesis begins by selection of compounds that are known as reagents or reactants. Various reaction types can be applied to these to synthesize the product, or an intermediate product. This requires mixing the compounds in a reaction vessel such as a chemical reactor or a simple roundbottom flask. Many reactions require some form of work-up procedure before the final product is isolated. The amount of product in a chemical synthesis is the reaction yield. Typically, chemical yields are expressed as a weight in grams (in a laboratory setting) or as a percentage of the total theoretical quantity of product that could be produced. A side reaction is an unwanted chemical reaction taking place that diminishes the yield of the desired product.

"Reacting" as used with regard to the method for synthesizing a compound library refers to contacting the educts under conditions that allow formation of the product, namely the conjugate molecule. Exemplary reaction conditions are described in the examples.

In various embodiments of the method, the conjugate molecule is further used in a reaction comprising a) a strong acid, preferably an acid having a pKₐ of less than 3; and/or b) a catalyst selected from the group consisting of copper, silver, gold, ruthenium, iron, osmium, cobalt, rhodium, iridium, nickel, palladium, platinum and derivatives thereof, preferably copper, silver, gold, ruthenium and derivatives thereof.

The strength of an acid refers to its ability or tendency to lose a proton (H⁺). A strong acid is one that completely ionizes (dissociates) in a solution. In water, one mole of a strong acid HA dissolves yielding one mole of H⁺ (as hydronium ion H₃O⁺) and one mole of the conjugate base, A⁻. Essentially, none of the non-ionized acid HA remains. Examples of strong acids are hydrochloric acid (HCl), hydroiodic acid (HI), hydrobromic acid (HBr), perchloric acid (HClO₄), nitric acid (HNO₃), trifluoroacetic acid, trichloroacetic acid, and sulfuric acid (H₂SO₄). In aqueous solution, each of these essentially ionizes 100%. In contrast, a weak acid only partially dissociates. Examples in water include carbonic acid (H₂CO₃) and acetic acid (CH₃COOH). At equilibrium, both the acid and the conjugate base are present in solution. Stronger acids have a larger acid dissociation constant (Kₐ) and a smaller logarithmic constant (pKₐ = -log Kₐ) than weaker acids.

The term "transition metal", as used herein, is a synonym for elements of the groups 3 to 12 of modern IUPAC numbering. Examples of transition metals are copper (Cu), silver (Ag), and gold (Au).

"Catalyst", as used herein, refers to any substance that increases the rate of a reaction without itself being consumed. In general, catalytic action is a chemical reaction between the catalyst and a reactant, forming chemical intermediates that are able to react more readily with each other or with another reactant, to form the desired end product. During the reaction between the chemical intermediates and the reactants, the catalyst is regenerated. The modes of reactions between the catalysts and the reactants vary widely. Typical of these reactions are acid-base reactions, oxidation-reduction reactions, formation of coordination complexes, and formation of free radicals.

In still various embodiments of the method, the nucleic acid moiety is elongated in an additional reaction step with pyrimidine and/or purine nucleotides after the conjugation step. As described above, the elongation of the nucleic acid molecule may be put into practice by using PCR and a primer nucleic acid molecule, which comprises a nucleic acid sequence that hybridizes with the adaptor nucleic acid and a nucleic acid sequence which encodes for a sequence that allows the individualization of the conjugate molecule.

It was surprisingly found that only purine nucleotides, but not pyrimidine nucleotides, are degraded under conditions used for the synthesis of heterocyclic chemical compounds (for example reactions involving the use of transition metals as catalysts or strong acids). However, the variety of chemical reactions carried out on the organic molecule is not limited to reactions that are only tolerated by pyrimidine nucleotides. This variety of chemical reactions may also comprise, but is not limited to amide synthesis, stepwise substitution of cyanuric chloride, protective group chemistry, amide bond formation, carbonyl reactions, such as synthesis of diamine-containing cyclic or linear compounds, aromatic substitutions, such as nucleophilic aromatic substitutions of reactive heteroaromatic halides, cross-coupling reactions, such as palladium-catalyzed synthesis of biaryl compounds, Diels-Alder reaction, benzimidazole formation, macrocycle synthesis, substitution with N-nucleophiles, nucleophilic substitution of reactive aliphatic halides, Cu(I)-catalyzed alkyne-azide cycloaddition, Horner-Wadsworth-Emmons reation with reactive phosphonium ylids, reduction of aromatic nitro groups and appendage of cyanuric chloride.

In a third aspect, the present invention relates to the use of a compound library of the present invention for screening a compound binding to a target molecule.

The term "screening", as used herein, means that a plurality of candidate compounds (this can be an amount of 10 or less compounds or up to 10000 compounds or more) can be screened in a single experiment for their ability to bind to a given target molecule. In various embodiments, 10 or more, 100 or more, 1000 or more, 1500 or more, 2000 or more, 5000 or more or 10000 or more of the candidate compounds are pooled in a single vessel and tested for their ability to bind to target molecule. Thus, said candidate compounds pooled in a single vessel and exposed to the target molecule compete for binding to said target molecule. Upon being brought into contact with the target molecule, the candidate compounds may bind to the target molecule or being washed out by one or more washing steps. Subsequently, the candidate compounds bound to the target molecule can be identified via their individual DNA label. Methods to identify candidate compounds based on their DNA label are described herein and are well-known in the art.

A "target molecule", as used herein, may be any molecule which can be bound by a binding molecule and may for example be a biological substance such as a biomolecule, complexes, cell fractions or cells. Preferably, a target molecule within the context of the present invention is a nucleic acid, e.g. DNA, or RNA molecule. Even more preferred are target molecules such as a peptide, a protein, a drug molecule, a small molecule, or a vitamin. In particular preferred embodiments of the present invention a "target molecule" may be a biomarker. A target molecule may also refer to a biological molecule that is either naturally present in a cell or subject or has been previously introduced into a cell or subject. A "target molecule" can be any polypeptide, lipid, nucleic acid, small molecule, saccharide or a vitamin of the cell. It may be located in the nucleus, nucleolus, cytoplasm, mitochondria, Golgi apparatus, endoplasmic reticulum or membrane of the cell. In various embodiments, the target molecules of the methods of the present invention are polypeptide molecules.

In various embodiments, the target molecule is a protein.

The term "protein", as used herein, relates to one or more associated polypeptides, wherein the polypeptides consist of amino acids coupled by peptide (amide) bonds. The term polypeptide refers to a polymeric compound comprised of covalently linked amino acid residues. The amino acids are preferably the 20 naturally occurring amino acids glycine, alanine, valine, leucine, isoleucine, phenylalanine, cysteine, methionine, proline, serine, threonine, glutamine, asparagine, aspartic acid, glutamic acid, histidine, lysine, arginine, tyrosine and tryptophan.

Suitable targets may include one or more of peptides, proteins (e.g., antibodies, affïbodies, or aptamers), nucleic acids (e.g., polynucleotides, DNA, RNA, or aptamers); polysaccharides (e.g., lectins or sugars), lipids, enzymes, enzyme substrates, ligands, receptors, antigens, or haptens. One or more of the aforementioned targets may be characteristic of particular cells, while other targets may be associated with a particular disease or condition. In some embodiments, targets in a tissue sample that may be detected and analyzed using the methods disclosed herein may include, but are not limited to, prognostic targets, hormone or hormone receptor targets, lymphoid targets, tumor targets, hematopoietic targets, cell cycle associated targets, neural tissue and tumor targets, or cluster differentiation targets.

Suitable examples of prognostic targets may include enzymatic targets such as galactosyl transferase II, neuron specific enolase, proton ATPase-2, or acid phosphatase.

Suitable examples of hormone or hormone receptor targets may include human chorionic gonadotropin (HCG), adrenocorticotropic hormone, carcinoembryonic antigen (CEA), prostate-specific antigen (PSA), estrogen receptor, progesterone receptor, androgen receptor, gClq-R/p33 complement receptor, IL-2 receptor, p75 neurotrophin receptor, PTH receptor, thyroid hormone receptor, or insulin receptor.

Suitable examples of hematopoietic targets may include CD45, CD34, CD133, HLA-DR, CD115, CD116, CD117, CD33, CD38, CD90, CD71, Ki67, Flt3, CD163, CD45RA, CD3, IgD, CD105, CD45, and also c-kit, - the receptor for stem cell factor.

Suitable examples of lymphoid targets may include alpha- 1- antichymotrypsin, alpha- 1 -antitrypsin, B cell target, bcl-2, bcl-6, B lymphocyte antigen 36 kD, BM1 (myeloid target), BM2 (myeloid target), galectin-3, granzyme B, HLA class I Antigen, HLA class II (DP) antigen, HLA class II (DQ) antigen, HLA class II (DR) antigen, human neutrophil defensins, immunoglobulin A, immunoglobulin D, immunoglobulin G, immunoglobulin M, kappa light chain, kappa light chain, lambda light chain, lymphocyte/histocyte antigen, macrophage target, muramidase (lysozyme), p80 anaplastic lymphoma kinase, plasma cell target, secretory leukocyte protease inhibitor, T cell antigen receptor (JOVI 1), T cell antigen receptor (JOVI 3), terminal deoxynucleotidyl transferase, or unclustered B cell target.

Suitable examples of tumour targets may include alpha fetoprotein, apolipoprotein D, BAG-1 (RAP46 protein), CA19-9 (sialyl lewisa), CA50 (carcinoma associated mucin antigen), CA1 25 (ovarian cancer antigen), CA242 (tumour associated mucin antigen), chromogranin A, clusterin (apolipoprotein J), epithelial membrane antigen, epithelial-related antigen, epithelial specific antigen, gross cystic disease fluid protein- 15, hepatocyte specific antigen, heregulin, human gastric mucin, human milk fat globule, MAGE-1, matrix metalloproteinases, melan A, melanoma target (HMB45), mesothelin, metallothionein, microphthalmia transcription factor (MITF), Muc-1 core glycoprotein. Muc-1 glycoprotein, Muc-2 glycoprotein, Muc- 5AC glycoprotein, Muc-6 glycoprotein, myeloperoxidase, Myf-3 (Rhabdomyosarcoma target), Myf-4 (Rhabdomyosarcoma target), MyoDl (Rhabdomyosarcoma target), myoglobin, nm23 protein, placental alkaline phosphatase, prealbumin, prostate specific antigen, prostatic acid phosphatase, prostatic inhibin peptide, PTEN, renal cell carcinoma target, small intestinal mucinous antigen, tetranectin, thyroid transcription factor- 1, tissue inhibitor of matrix metalloproteinase 1, tissue inhibitor of matrix metalloproteinase 2, tyrosinase, tyrosinase-related protein- 1, villin, or von Willebrand factor.

Suitable examples of cell cycle associated targets may include apoptosis protease activating factor- 1, bcl-w , bcl-x, bromodeoxyuridine, CAK (cdk-activating kinase), cellular apoptosis susceptibility protein (CAS), caspase 2, caspase 8, CPP32 (caspase-3), CPP32 (caspase-3), cyclin dependent kinases, cyclin A, cyclin Bl, cyclin D1, cyclin D2, cyclin D3, cyclin E, cyclin G, DNA fragmentation factor (N-terminus), Fas (CD95), Fas-associated death domain protein, Fas ligand, Fen-1, IPO-38, McI-1, minichromosome maintenance proteins, mismatch repair protein (MSH2), poly (ADP- Ribose) polymerase, proliferating cell nuclear antigen, p16 protein, p27 protein, p34cdc2, p57 protein (Kip2), pi 05 protein, Stat 1 alpha, topoisomerase I, topoisomerase II alpha, topoisomerase III alpha, or topoisomerase II beta.

Suitable examples of neural tissue and tumor targets may include alpha B crystallin, alpha-internexin, alpha synuclein, amyloid precursor protein, beta amyloid, calbindin, choline acetyltransferase, excitatory amino acid transporter 1, GAP43, glial fibrillary acidic protein, glutamate receptor 2, myelin basic protein, nerve growth factor receptor (gp75), neuroblastoma target, neurofilament 68 kD, neurofilament 160 kD, neurofilament 200 kD, neuron specific enolase, nicotinic acetylcholine receptor alpha4, nicotinic acetylcholine receptor beta2, peripherin, protein gene product 9, S- 100 protein, serotonin, SNAP-25, synapsin I, synaptophysin, tau, tryptophan hydroxylase, tyrosine hydroxylase, or ubiquitin.

Suitable examples of cluster differentiation targets may include CDIa, CDIb, CDIc, CDId, CDIe, CD2, CD3delta, CD3epsilon, CD3gamma, CD4, CD5, CD6, CD7, CD8alpha, CD8beta, CD9, CDIO, CD1 Ia, CD1 Ib, CD1 Ic, CDw12, CD13, CD14, CD15, CD15s, CD16a, CD16b, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42a, CD42b, CD42c, CD42d, CD43, CD44, CD44R, CD45, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD65s, CD66a, CD66b, CD66c, CD66d, CD66e, CD66f, CD68, CD69, CD70, CD71, CD72, CD73, CD74, CDw75, CDw76, CD77, CD79a, CD79b, CD80, CD81, CD82, CD83, CD84, CD85, CD86, CD87, CD88, CD89, CD90, CD91, CDw92, CDw93, CD94, CD95, CD96, CD97, CD98, CD99, CD100, CD101, CD102, CD103, CD104, CD105, CD106, CD107a, CD107b, CDwl08, CD109, CD1 14, CD1 15, CD1 16, CD1 17, CDwI 19, CD120a, CD120b, CD121a, CDw121b, CD122, CD123, CD124, CDw125, CD126, CD127, CDw128a, CDw128b, CD130, CDw131, CD132, CD134, CD135, CDw136, CDw137, CD138, CD139, CD140a, CD140b, CD141, CD142, CD143, CD144, CDw145, CD146, CD147, CD148, CDw149, CDw150, CD151, CD152, CD153, CD154, CD155, CD156, CD157, CD158a, CD158b, CD161, CD162, CD163, CD 164, CD 165, CD 166, and TCR-zeta.

Other suitable prognostic targets hormone or hormone receptor targets lymphoid targets tumor targets cell cycle associated targets neural tissue and tumor targets include centromere protein-F (CENP-F), giantin, involucrin, lamin A&C (XB 10), LAP-70, mucin, nuclear pore complex proteins, pi 80 lamellar body protein, ran, cathepsin D, Ps2 protein.

The term "contacting", as used herein in the context of conjugate molecules and target molecules, refers generally to providing access of one component, reagent, analyte or sample to another. For example, contacting can involve mixing a solution comprising a conjugate molecule and a target molecule. The solution comprising one component, reagent, analyte or sample may also comprise another component or reagent, such as dimethyl sulfoxide (DMSO) or a detergent, which facilitates mixing, interaction, uptake, or other physical or chemical phenomenon advantageous to the contact between components, reagents, analytes and/or samples.

The term "identify", as used herein in the context of screening, relates to the recognition of a conjugate molecule that has the ability to completely or partly revert molecular changes of a cell induced by pathological conditions or to inhibit or activate enzymatic functions.

The term "sequence", as used herein, relates to the primary nucleotide sequence of nucleic acid molecules or the primary amino acid sequence of a protein.

### EXAMPLES

### Example 1: Coupling of (amino) acids to 5'-aminolinker-modified DNA bound to controlled pore glass (CPG) solid support

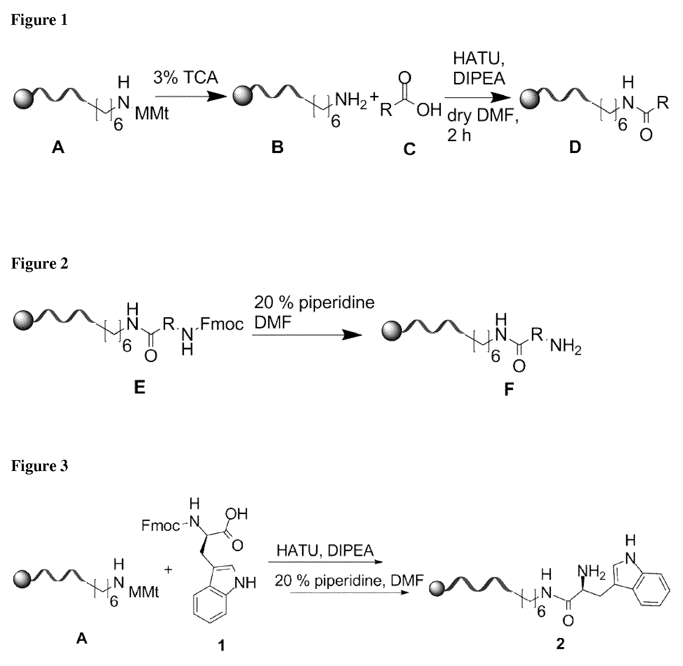

The MMt-protective group of 3'-5'-dTdTdTdTdTdT-5'-(6-aminohexyl)-hydrogen phosphate **A** bound to 1000 Å controlled pore glass (CPG) solid support was removed by addition of 3 % trichloroacetic acid in dry DCM. A yellow color indicated successful removal of the protective group. After that, the CPG was washed three times with each 200 µL of 1 % TEA in AcCN, DMF, MeOH, ACN and DCM. Then, the 3'-5'-hexathymidine-5'-aminolinker DNA **B** bound to 1000 Å controlled pore glass (CPG) solid support, an (amino) acid C, and a coupling reagent were dried *in vacuo* for 15 min. Then, stock solutions of all reactants in DMF were prepared immediately before the reactions were started. An amino acid C (100 eq) was coupled to the aminolinker-modified DNA **B** using HATU (100 eq) as coupling reagent and DIPEA (250 eq) as base. To a 15 µL of a solution of a carboxylic acid **C** in *dry* DMF were added 15 µL of HATU dissolved in *dry* DMF and DIPEA (250 eq.) was added. The carboxylic acid C was then activated for 20 min at room temperature in a shaker. Afterwards, the solid support-bound DNA **B** suspended in DMF (15 µL) was added. The reaction tube was sealed and the reaction mixture was shaken at room temperature for 2 hours. Then, the CPG containing the DNA-conjugate **D** was filtered off using a filter column and washed subsequently with each 3 x 200 µL of DMF, MeOH, MeCN and DCM. The filter with the CPG-bound product was then dried *in vacuo* for 15 min. 20-30 nM of the DNA were deprotected and removed from the CPG by treatment with AMA (AMA= aqueous ammonia (30 %)/ aqueous methylamine (40 %) 1:1vol/vol) and analyzed by HPLC to evaluate the coupling efficiency. Then, 20 µL of 1M Tris-buffer was added, the mixture was dried in a SpeedVac, re-dissolved in 100 µL of distilled water and analyzed by HPLC on a Gemini 5u C18 11O A column; 100*10.0 mm with a gradient of aqueous triethylammonium acetate buffer (100 mM, pH= 8) and methanol (20:80 - 70:30 of methanol over 20 min). For further reaction, the solid support containing compound **D** was reacted three times with each 200 µL of a 1:1 solution of capping solutions A and B (A: 800 µL dry THF/ 100 µL acetic acid anhydride/ 100 µL pyridine; B: 900 µL dry THF/ 100 µL *N*-methylimidazole) for each 30 seconds. This step capped unreacted amines, and it was followed by washing of solid support three times with each of 200 µL of DMF, MeOH, ACN and DCM. The CPGs were finally washed again with each 3 x 200 µL of DMF, MeOH, MeCN and DCM (see Figure 1).

### Example 2: Removal of the Fmoc-protective group from DNA-amino acid conjugates bound to controlled pore glass (CPG) solid support

The CPG containing an Fmoc-protected 3'-5'-hexathymidine-5'-amino acid conjugate **E** was washed with each 3 x 200 µL of DMF, MeOH, MeCN and DCM. Fmoc-deprotection was performed with 20 % piperidine in DMF (100 µL) for 15 min at room temperature. The CPG containing the deprotected conjugate **F** was filtered off and washed with each 3 x 200 µL of DMF, MeOH, MeCN and DCM. The CPG was then dried *in vacuo* for 15 min (see Figure 2).

### Example 3: Synthesis of 3'-5'-hexathymidine-5'-β-carboline-conjugates by Pictet-Spengler reaction

### a) Synthesis of DNA-tryptophane conjugates

The amide coupling of Fmoc-tryptophane **1** to 3'-5'-hexathymidine-5'-aminolinker DNA **B** was performed as described above in Example 1 using 3'-5'-hexathymidine-5'-aminolinker DNA **B** on 1000 Å CPG (60 nM), Fmoc-Trp-OH (6 µmol, 2.6 mg), HATU (6 µmol, 2.3 mg), and 2.6 µL DIPEA. The removal of the Fmoc-protective group yielding 3'-5'-dTdTdTdTdTdT-5'-((*S*)-6-(2-amino-3-(1*H-*indol-3-yl)propanamido)hexyl)hydrogen phosphate **2** was done according to Example 2 (see Figure 3).

### b) Pictet-Spengler reaction

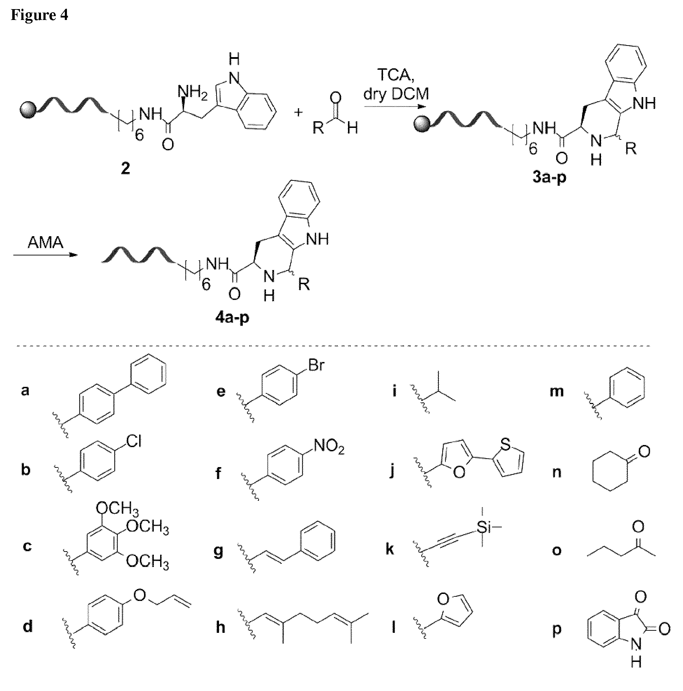

To a suspension of the DNA-Tryp-NH₂ conjugate bound to CPG (ca. 30 nM, 1.1 mg) in a solution of a suitable catalyst (trichloroacetic acid (TCA), 0.03 M; trifluoroacetic acid (TFA), 0.03 M; diphenyl hydrogen phosphate (DHP) 0.03 M) in 45 µl of a suitable solvent (dry DCM, MeCN, DCE, PhMe) was added 100 eq (3 µmol) of an aldehyde or ketone of choice (e.g. biphenyl-4-carboxaldehyde a, 3 µmol, 0.55 mg) (see Figure 4).

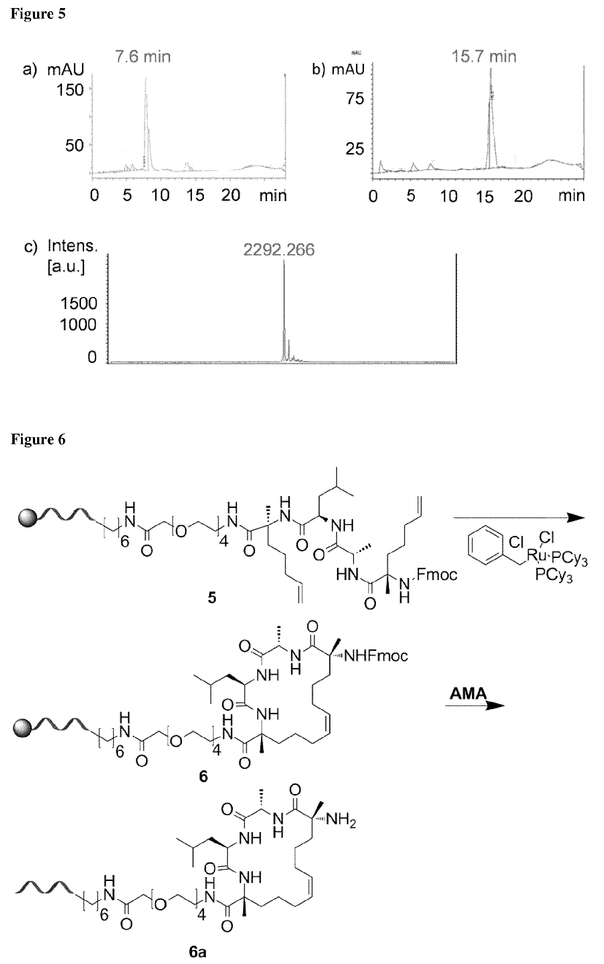

The reaction was shaken at room temperature for 16 h, then, the DNA was filtered off, washed successively with DMF (3 x 200 µl), MeCN (3 x 200 µl), MeOH (3 x 200 µl) and DCM (3 x 200 µl), and dried *in vacuo* for 15 min. The DNA was deprotected and removed by treatment with AMA (AMA= aqueous ammonia (30 %)/ aqueous methylamine (40 %) 1:1 vol/vol). Then, 20 µL of 1M Tris-buffer was added, the mixture was dried in a SpeedVac, re-dissolved in 100 µL of distilled water and purified by HPLC on a Gemini Su C18 110 A column; 100*10.0 mm with a gradient of aqueous triethylammonium acetate buffer (100 mM, pH= 8) and methanol (20:80 - 70:30 of methanol over 19 min). HPLC purification and MALDI-MS analysis of 3'-5'-dTdTdTdTdTdT-5'-(6-((*R*)-1-([1,1'-biphenyl]-4-yl)-2,3,4,9-tetrahydro-1*H*-pyrido[3,4-b]indole-3-carboxamido)hexyl)hydrogen phosphate **4a** are shown in Figure 5.

### Example 4: Ruthenium-catalyzed ring-closing metathesis to 3'-5'-hexathymidine-peptide conjugate 6a

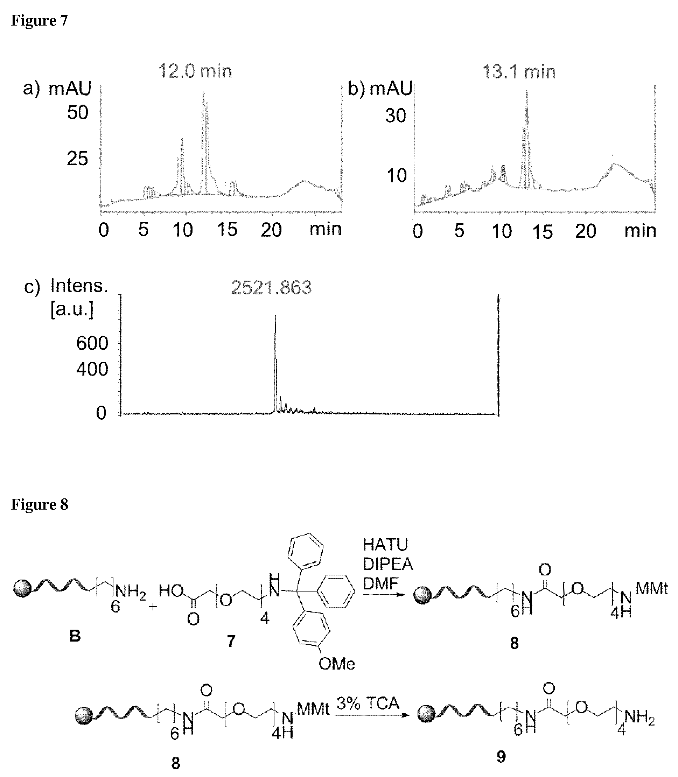

The synthesis of peptide-DNA conjugates was performed by Fmoc-solid-phase peptide synthesis (**SPPS**) by iterative application of the protocols of Examples 1 and 2 making use of amino acids in the following order: 1. (S)-*N*-fmoc-alpha-4-pentenylalanine (25 µmol, 9.5 mg); 2. *N*-fmoc-L-leucine (22 µmol, 7.8 mg); 3. *N*-fmoc-L-alanine (19 µmol, 5.9 mg); 4. (*R*)-*N*-fmoc-alpha-4-pentenyl alanine (16 µmol, 6.1mg). Crosslinking of unnatural olefinic amino acids was performed by ring closing metathesis (RCM) using the Grubbs first generation catalyst bis(tricyclohexylphosphine)benzylidine ruthenium(IV) dichloride (4.9 µmol, 4 mg) dissolved in 1 ml of dry dichloromethane (DCM) three times for 2 h at room temperature (100 µl of the solution were used for each coupling) (Figure 6). After each coupling, the DNA was washed with DCM (3 x 200 µl), the CPG was then dried *in vacuo* for 5 min and the next coupling was performed. Subsequent to the three couplings, the DNA was washed successively with DMF (3 x 200 µl), ACN (3 x 200 µl), MeOH (3 x 200 µl) and DCM (3 x 200 µl). The filter carrying CPGs was then dried *in vacuo* for 15 min. The DNA-macrocycle conjugate was deprotected and removed from the CPG by treatment with AMA (AMA= aqueous ammonia (30 %)/ aqueous methylamine (40 %) 1:1 vol/vol) . Then, 20 µL of 1M Tris-buffer was added, the mixture was dried in a SpeedVac, re-dissolved in 100 µL of distilled water and purified by HPLC on a Gemini Su C18 110 A column; 100*10.0 mm with a gradient of aqueous triethylammonium acetate buffer (100 mM, pH=8) and methanol (20:80 - 70:30 of methanol over 20 min) (see Figure 7). HPLC purification and MALDI-MS analysis of 3'-5'-dTdTdTdTdTdT-5'-(6-(2-(2-((2*S*,*5*R,8*R*,17*R*,Z)-17-amino-5-isobutyl-2,8,17-trimethyl-3,6,18-trioxo-1,4,7-triazacyclooctadec-12-ene-8-carboxamido)ethoxy)acetamido)hexyl)hydrogen phosphate **6a** are shown in Figure 7.

### Example 5a: Gold-catalyzed cycloaddition to 3'-5'-hexathymidine-6-oxa-1,2-diazaspiro[4.4]nonane-conjugates

### a) Synthesis of hexathymidine-5'-PEG linker conjugate 9

The synthesis of the 3'-5'-hexathymidine-conjugate **9** was done according to general protocol of Example 1 making use of 400 nmol MMt-protected 3'-5'-hexathymidine-5'-aminolinker DNA **B** attached to solid support (ca. 14.4 mg of CPG), PEG-linker **7** (21.5 mg, 40 µmol, 100 eq.) dissolved in 120 µL of dry DMF, HATU (15.2 mg,40 µmol, 100 eq.) dissolved in 120 µL of dry DMF, and DIPEA (17.3 µL, 100 µmol, 250 eq.). To remove the MMt group of the PEG linker, the solid support was treated with 3 % trichloroacetic acid in dry DCM yielding 3'-5'-dTdTdTdTdTdT-5'-(1-amino-15-oxo-3,6,9,12-tetraoxa-16-azadocosan-22-yl)hydrogen phosphate (**9**, Figure 8). For purpose of analysis, 20 nmol of DNA-conjugate **9** (ca. 0.72 mg) were deprotected and removed from the CPG with 500 µL of a 1:1 solution of conc. aq. NH₃ and conc. aq. MeNH₂ for 30 minutes at room temperature. Then, 20 µL of 1M Tris-buffer was added, the mixture was dried in a SpeedVac, re-dissolved in 100 µL of distilled water and analyzed by HPLC on a Gemini 5u C18 110A column; 100*10.0 mm with a gradient of aqueous triethylammonium acetate buffer (100 mM, pH= 8) and methanol (20:80 - 70:30 of methanol over 20 min).

### b) Synthesis of 3'-5'-hexathymidine-aldehyde conjugate 11

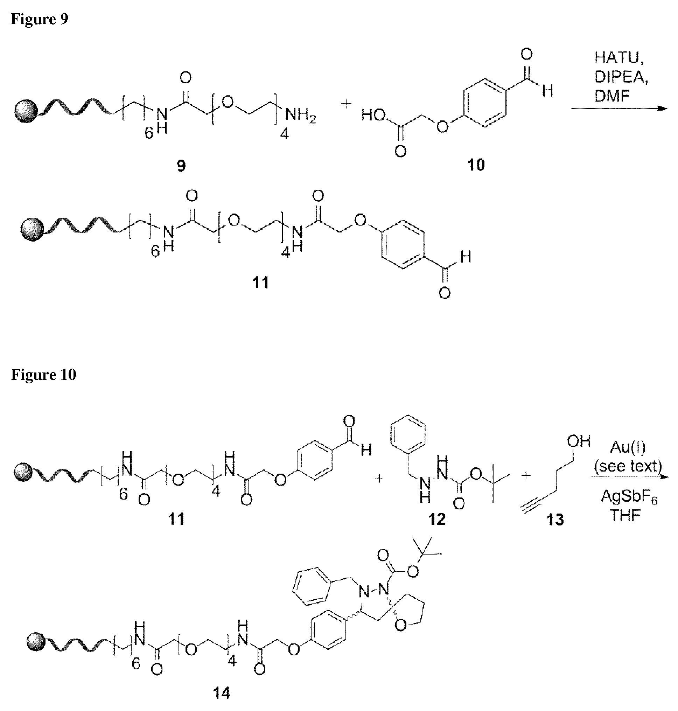

The synthesis of the hexathymidine-5'-conjugate **11** was done according to general protocol of Example 1 making use of 360 nmol of conjugate **9** attached to the solid support (ca. 13 mg of CPG), 2-(4-formylphenoxy)acetic acid 10 (6.5 mg, 36 µmol, 100 eq.) dissolved in 108 µL of dry DMF, HATU(13,7 mg, 36 µ mal, 100 eq.) dissolved in 108 µL of dry DMF, and DIPEA (15,6 µL, 90 µmal, 250 eq.) (Figure 9). For purpose of analysis, 20 nmol of 3'-5'-dTdTdTdTdTdT-5'-(1-(4-formylphenoxy)-2,18-dioxo-6,9,12,15-tetraoxa-3,19-diazapentacosan-25-yl)-hydrogen phosphate **11** (ca. 0,72 mg) were deprotected and removed from the CPG with 500 µL of a 1:1solution of conc. aq. NH₃ and conc. aq. MeNH₂ for 30 minutes at room temperature. Then, 20 µL of 1M Tris-buffer was added, the mixture was dried in a SpeedVac, re-dissolved in 100 µL of distilled water and analyzed by HPLC on Gemini 5u C18 110 A column; 100*10.0 mm with a gradient of aqueous triethylammonium acetate buffer (100 mM, pH= 8) and methanol (20:80 - 70:30 % of methanol over 19 min).

### c) Gold-catalyzed cycloaddition reaction to 3'-5'-hexathymidine-6-oxa-1,2-diazaspiro[4.4]nonane-conjugate 14

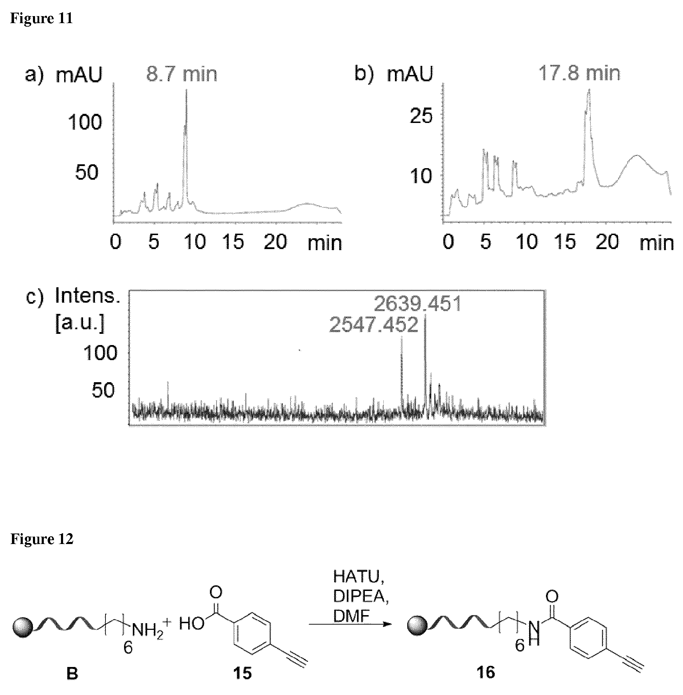

*tert*-Butyl-2-benzylhydrazine-1-carboxylate **12** (2,36 mg, 10 µmol, 500 eq) dissolved in 15 µL of dry THF, pent-4-yn-1-ol **13** (1.98 µL, 20 µmol, 100 eq), and chloro[tris(2,4-di-*tert-*butylphenyl)phosphite]gold (4.4 mg, 5 µmol, 250 eq) mixed with silver hexafluoroantimonate (1.72 mg, 5 µmol, 250 eq) in 15 µL of dry THF, were added to 20 nmol of polypyrimidine ONA-conjugate **11** (ca. 0.72 mg) suspended in 15 µL of dry THF (Figure 10). The reaction mixture was shaken at room temperature for overnight to furnish *tert*-butyl 3-benzyl-4-(24-(hydroxyl)(5'-(3'-5'-dTdTdTdTdTdT))phosphoryl)oxy)-2,17-dioxo-6,9,12,15-tetraoxa-3,18-diazatetracosyl)oxy)phenyl)-1,2,3-oxadiazocane-2-carboxylate **14**. After that, the solid support was washed three times with each 200 µL of DMF, MeOH, ACN and DCM. Then it was treated with 500 µL of 1:1 solution of conc.aq. NH₃ and conc. aq. MeNH₂ for 30 minutes at room temperature in order to remove it from CPG. Then, 20 µL of 1M Tris-buffer was added, the mixture was dried in a SpeedVac, re-dissolved in 100 µL of distilled water and purified by HPLC on Gemini 5u C18 110A column; 100*10.0 mm with a gradient of aqueous triethylammonium acetate buffer (100 mM, pH= 8) and methanol (20:80 - 70:30 of methanol over 19 min) HPLC purification and MALDI-MS analysis of the synthesized compound **14** are shown in Figure 11.

### Example 5b: Synthesis of 3'-5'-hexathymidine-2,3-dihydro-1H-pyrazole-conjugates

### a) Synthesis of hexathymidine-5'-alkyne conjugate 16

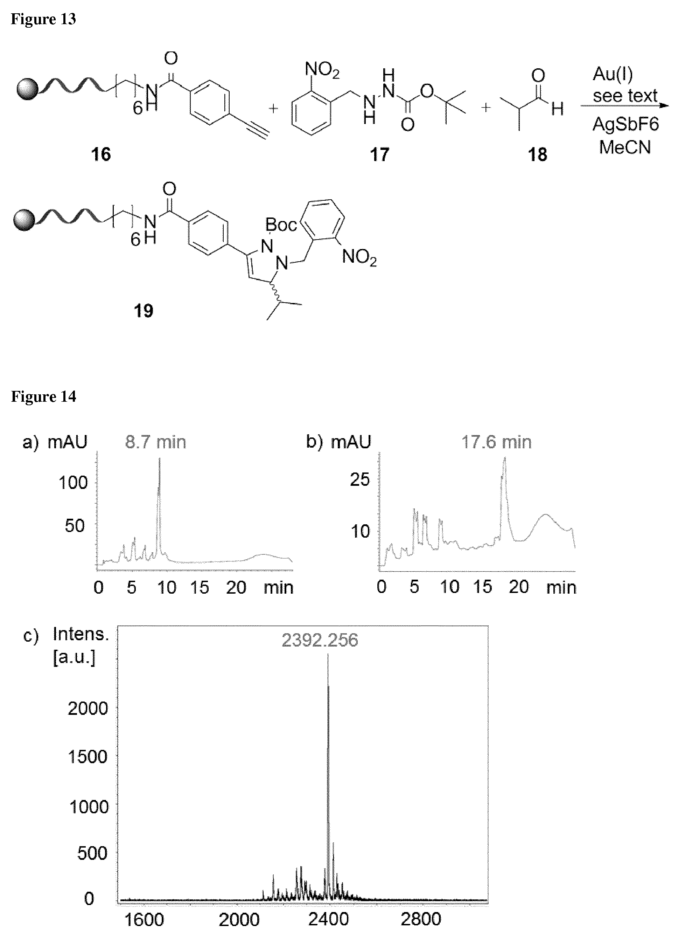

The synthesis of the DNA-alkyne conjugate **16** (Figure 12) was done according to general procedure 1 making use of 350 nmol 5'-aminolinker-modified 3'-5'-hexathymidine **B** attached to solid support (ca. 13 mg of CPG), 4-ethynylbenzoic acid 15 (5.1 mg, 35 µmol, 100 eq.) dissolved in 105 µL of dry DMF, HATU (13.3 mg, 35 µmol, 100 eq.) dissolved in 105 µL of dry DMF, and DIPEA (15.1 µL, 87.5 µmol, 250 eq.). For purpose of analysis (Figure **14a**), 30 nmol of 3'-5'-dTdTdTdTdTdT-5'-(6-(4-ethynylbenzamido)hexyl)hydrogen phosphate **16** (ca. 1.08 mg) were deprotected and removed from the CPG with 500 µL of a 1:1 solution of conc. aq. NH₃ and conc. aq. MeNH₂ for 30 minutes at room temperature. Then, 20 µL of 1 M Tris-buffer was added, the mixture was dried in a SpeedVac, re-dissolved in 100 µL of distilled water and analyzed by HPLC on Gemini 5u C18 110A column; 100*10.0 mm with a gradient of aqueous triethylammonium acetate buffer (100 mM, pH= 8) and methanol (20:80 - 70:30 of methanol over 19 min).

### b) Gold-catalyzed synthesis of 3'-5'-hexathymidine-2,3-dihydro-1H-pyrazole conjugate 19

*tert*-Butyl 2-(2-nitrobenzyl)hydrazinecarboxylate **17** (10.7 mg, 40 µmol, 1000 eq) dissolved in 45 µL of dry AcCN, isobutyraldehyde **18** (3.7 µL, 40 µmol, 1000 eq), and chloro[tris(2,4-di-*tert-*butylphenyl)phosphite]gold (8.8 mg, 10 µmol, 250 eq) mixed with silver hexafluoroantimonate (3.4 mg, 10 µmol, 250 eq) in 45 µL of dry MeCN, were added to 40 nmol of DNA-conjugate **16** (ca. 1.4 mg). The reaction mixture was shaken at 50 °C for overnight to yield *tert*-butyl 3-benzyl-4-(4-((24-hydroxy(5'-(3'-5'-dTdTdTdTdTdT))phosphoryl)oxy)-2,17-dioxo-6,9,12,15-tetraoxa-3,18-diazatetracosyl)oxy)phenyl)-1,2,3-oxadiazocane-2-carboxylate **19** (Figure 13). After that, the solid support was washed three times with each 200 µL of 0.1 M EDTA, DMF, MeOH, MeCN and DCM. Then, it was treated with 500 µL of 1:1 solution of conc. aq. NH₃ and conc. aq. MeNH₂ for 30 minutes at room temperature in order to remove it from CPG. 20 µL of 1M Tris-buffer was added, the mixture was dried in a SpeedVac, re-dissolved in 100 µL of distilled water and purified by HPLC on Gemini 5u C18 110A column; 100*10.0 mm with a gradient of aqueous triethylammonium acetate buffer (100 mM, pH= 8) and methanol (20:80 - 70:30 % of methanol over 19 min). HPLC purification and MALDI-MS analysis of the newly synthesized compound **19** are shown in Figures 14b and 14c.

### Example 6: Cycloaddition reactions yielding 3'-5'-hexathymidine-5'-spirooxindole-conjugates

### a) Synthesis of oxindole 20 (tert-butyl 4-(3-benzylidene-2-oxoindolin-1-yl)butanoate)

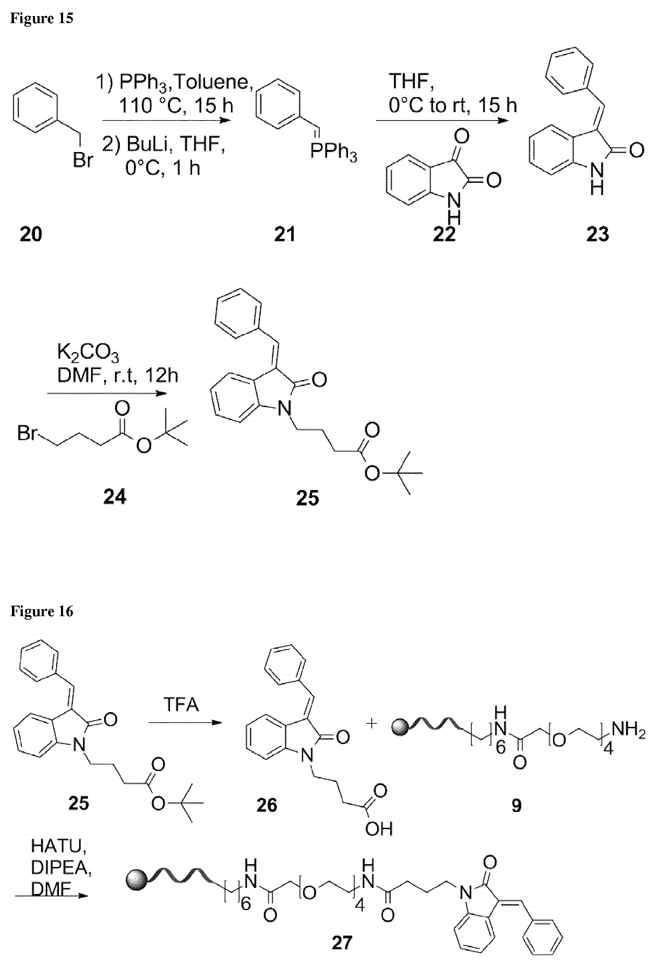

To triphenylphosphine (1.3 g, 5 mmol) in 10 ml of toluene was added benzyl bromide **20** (0.6 ml, 5 mmol) via syringe, the reaction was heated at 110 °C for 15 h, then the mixture was cooled to room temperature and the solid formed was isolated by suction filtration, washed with diethyl ether (3 x 5 ml) and dried *in vacuo* to give a white solid. Under an atmosphere of argon, 10 ml of anhydrous THF was added and the solution was cooled to 0 °C. A solution of butyl lithium (2.5 M in hexanes, 2 ml, 5 mmol) was added dropwise via syringe and the reaction was allowed to stir for 1h in order to form **21.** A solution of isatin **22** (0.75 g, 5 mmol) in dry THF (30 ml) was added dropwise via syringe over 20 min at 0 °C and the resulting solution was allowed to stir at room temperature for 15 h. The reaction mixture was then poured into a saturated solution of NH₄Cl (35 ml) at 0 °C and extracted with dichloromethane (3 x 25 mL). The combined organic layers were dried over anhydrous MgSO₄, filtered and concentrated *in vacuo* to afford a residue containing a E/Z mixture (ca. 4:1) of product 3-benzylideneindolin-2-one **23** that was separated by flash column chromatography, eluting with 3:7 EtOAc in hexanes, to give (E)-**23** (605 mg, 62%) and (Z)-**23** (155 mg, 15%) as yellow solids. To a solution of 3-benzylideneindolin-2-one **23** (130 mg, 0.6 mmol) in 10 ml of DMF was added potassium carbonate (207 mg, 1.5 mmol). The reaction was stirred at room temperature for 15 minutes. *tert-*Butyl-4-bromobutyrate **24** (212 µL, 1.2 mmol) was then added. The reaction was stirred at room temperature overnight, concentrated *in vacuo*, dissolved in EtOAc and washed with H₂O. The layers were separated and the aqueous layer was extracted with additional EtOAc. The combined organic phase was dried over Na₂SO₄, filtered and concentrated *in vacuo*. The crude product was purified by silica gel column chromatography, eluting with 3:7 EtOAc in petroleum ether, to give the product *tert*-butyl 4-(3-benzylidene-2 -oxoindolin-1-yl)butanoate **25** as an orange solid (83%, 180 mg) (Figure 15).

### b) Synthesis of the 3'-5'-hexathymidine-5'-oxindole conjugate 22

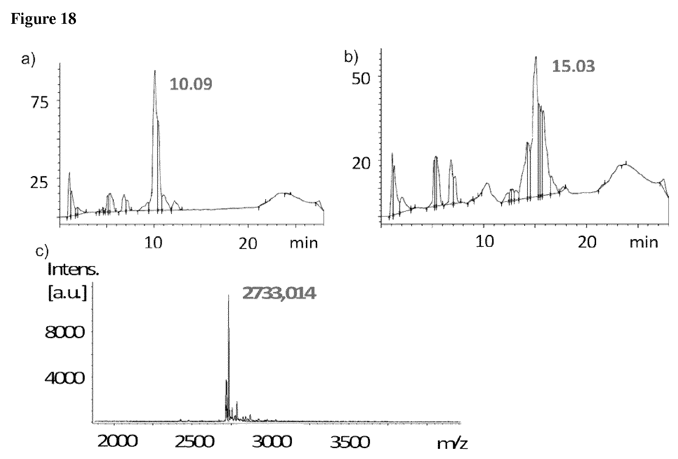

After the deprotection of *tert*-butyl-4-(3-benzylidene-2-oxoindolin-1-yl)butanoate **25** with TFA, the synthesis of 3'-5'-dTdTdTdTdTdT-5'-((E)-1-(3-benzylidene-2-oxoindolin-1-yl)-4,19-dioxo-8,11,14,17-tetraoxa-5,20-diazahexacosan-26-yl)hydrogen phosphate **27** was done according to general protocol of Example 1 making use of 400 nmol hexathymidine DNA-PEG linker conjugate **9** attached to the solid support, (*E*)-4-(3-benzylidene-2-oxoindolin-1-yl)butanoic acid **26** (12.3 mg, 40 µmol, 100 eq.) dissolved in 100 µL of dry DMF, HATU (15.2 mg, 49 µmol, 100 eq.) dissolved in 100 µL of dry DMF, and DIPEA (17.4 µL, 100 µmol, 250 eq.) (Figure 16). For purpose of analysis 20 nmol of DNA-conjugate **27** were deprotected and removed from the CPG with 500 µL of a 1:1 solution of conc. aq. NH₃ and conc. aq. MeNH₂ for 30 minutes at room temperature. Then, 20 µL of 1M Tris-buffer was added, the mixture was dried in a SpeedVac, re-dissolved in 100 µL of distilled water and analyzed by HPLC on Gemini 5u C18 110A column; 100*10.0 mm with a gradient of aqueous triethylammanium acetate buffer (100 mM, pH= 8) and methanol (20:80 - 70:30 of methanol over 19 min, Figure 18a).

### c) Cu(I)-catalyzed synthesis of hexathymidine-5'-spirooxindole [methyl 1-(4-amino-4- oxobutyl)-2'-(4-bromophenyl)-2-oxo-4'-phenylspiro[indoline-3,3'-pyrrolidine]-5'-carboxylate] conjugate 29 via (3+2) cycloaddition

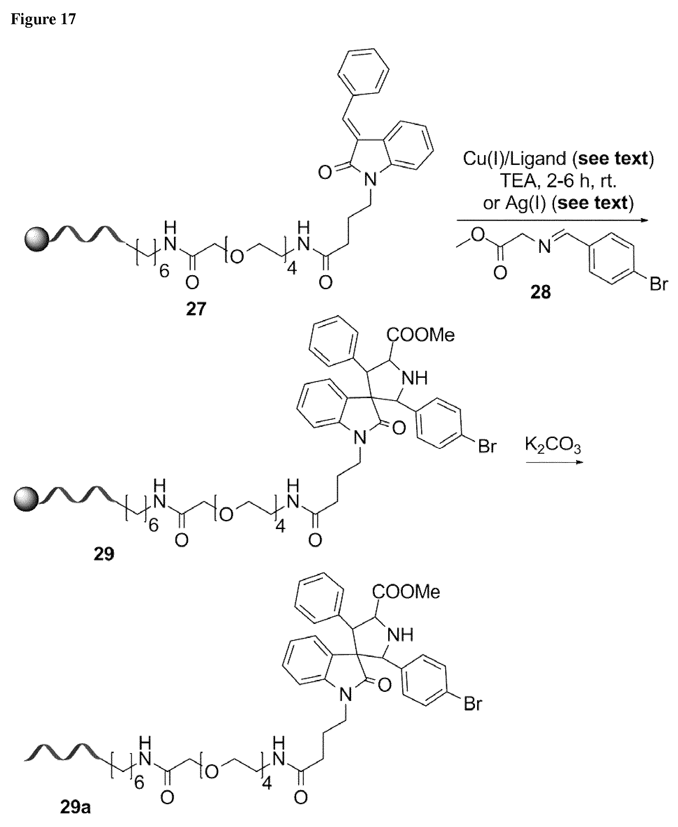

The catalyst [Cu(CH₃CN)_{4]}PF₆ (0.05 mg, 0.12 µmol, 5 eq) and one of the ligands (RP,R'P)-1,1'-bis[(S)-α-(dimethylamino)benzyl]-2,2'-bis(diphenylphosphino)ferrocene (0.4 mg, 0.5 µmol, 20 eq) or [tris[(1-benzyl-1*H*-1,2,3-triazol-4-yl) methyl]amine) (0.26 mg, 0.5 µmol, 20 eq) were dissolved in 60 µl of DCM and mixed with methyl 2-((4-bromobenzylidene)amino)acetate **28** (0.76 mg, 3 µmol, 100 eq) in 30 µl of DCM and triethylamine (0.37 µl, 2.5 µmol, 100 eq), the mixture was added to 30 nmol of DNA-conjugate **27** (ca. 1 mg) and shaken at room temperature for 4 h to yield methyl 2'-(4-bromophenyl)-1-(26-(hydroxy-(5'-(3'-5'-dTdTdTdTdTdT))phosphoryl)oxy)-4,19-dioxo-8,11,14,17-tetraoxa-5,20-diazahexacosyl)-2-oxo-4'-phenylspiro[indoline-3,3'-pyrrolidine]-5'-carboxylate (**29**, Figure 17). After that, the solid support was washed three times with each 200 µL of DMF, MeOH, ACN and DCM. Then it was treated with 500 µL of a solution of K₂CO₃ in methanol for 30 minutes at room temperature. The solution was neutralized with aq. acetic acid, 20 µL of 1M Tris-buffer was added, the mixture was dried in a SpeedVac, re-dissolved in 100 µL of distilled water and purified by HPLC on Gemini 5u C18 110 A column; 100*10.0 mm with a gradient of aqueous triethylammonium acetate buffer (100 mM, pH= 8) and methanol (20:80 - 70:30 % of methanol over 19 min) and analyzed by MALD1-TOF analysis (Figure 18b and 18c).

### d) Ag-catalyzed (3+2) cycloaddition yielding hexathymidine-5'-spirooxindole conjugate 29

To a solution of catalyst AgOAc (0.04 mg, 0.25 µmol, 10 eq) and triethylamine (0.37 µl, 2.5 µmol, 100 eq) in 60 µl of DCM or THF was added methyl 2-((4-bromobenzylidene)amino)acetate **28** (0,76 mg, 3 µmol, 100 eq) in 30 µl of DCM or THF, the mixture was added to 30 nmol of DNA-conjugate **27** (ca. 1 mg) shaken at room temperature for 4 h to furnish methyl 2'-(4-bromophenyl)-1-(26-(hydroxy-(5'-(3'-5'-dTdTdTdTdTdT))phosphoryl)oxy)-4,19-dioxo-8,11,14,17-tetraoxa-5,20-diazahexacosyl)-2-oxo-4'-phenylspiro[indoline-3,3'-pyrrolidine]-5'-carboxylate (**29**, Figure 17). After that, the solid support was washed three times with each 200 µL of DMF, MeOH, ACN and DCM. Then it was treated with 500 µL of a solution of K₂CO₃ in methanol for 30 minutes at room temperature. The solution was neutralized with aq. acetic acid. Then, 20 µL of 1M Tris-buffer was added, the mixture was dried in a SpeedVac, re-dissolved in 100 µL of distilled water and purified by HPLC on Gemini Su C18 110A column; 100*10.0 mm with a gradient of aqueous triethylammonium acetate buffer (100 mM, pH= 8) and methanol (20:80 - 70:30 % of methanol over 19 min, Figure 18b), and analyzed by MALD1-TOF analysis.

### Example 7: Encoding of hexaT-adapter adaptor nucleic acid-small molecule conjugates by T4 ligase

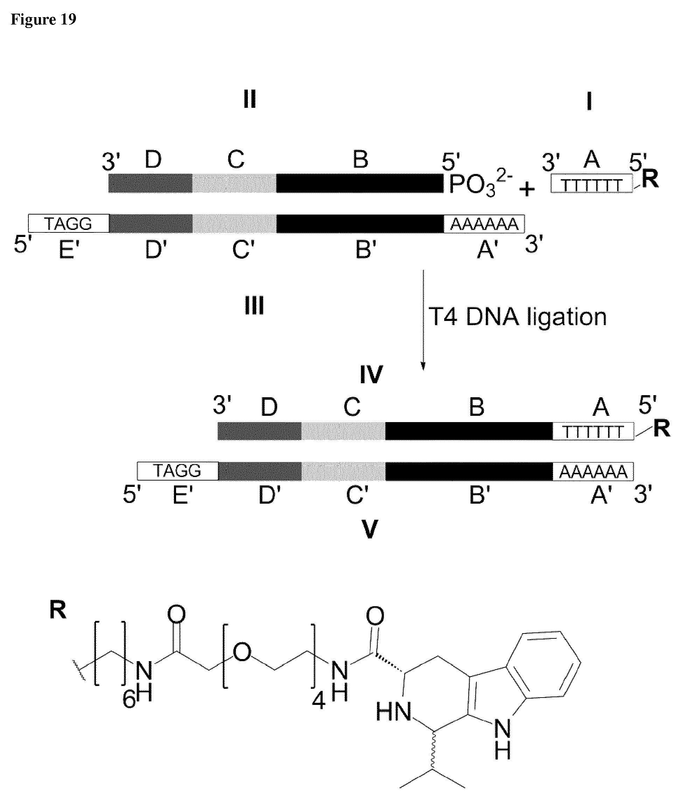

Coding oligonucleotides were phosphorylated at the 5'-end with T4 polynucleotide kinase (T4 PNK, Thermo Scientific). The phosphorylation reactions for the first ligation were performed in a total volume of 20 µl using 280 pmol of the coding oligonucleotides (e.g. oligonucleotide II (Figure 19)).

**Table 1: reaction mixture of PNK reaction for the first ligation**

| | |
|---|---|
| oligonucleotide **II** | 280 pmol (2,8 µl) |
| 10X reaction buffer A (500 mM Tris-HCl; pH 7.6 at 25°C, 100 mM MgCl2, 50 mM DTT, 1 mM spermidine) | 2 µl |
| ATP, 10 mM | 2 µl |
| T4 Polynucleotide Kinase | 1 µl (10 u) |
| water, nuclease-free | to 20 µl |

The PNK reaction mixtures were mixed thoroughly, spinned briefly and incubated at 37°C for 20 minutes. The inactivation of PNK was done by incubating the samples at 75°C for 10 minutes. Oligonucleotides **I** (adapter nucleic acid-beta-carboline conjugate), **II** (phosphorylated) and **III** were hybridized at 65°C for 10 minutes and slowly cooled down to room temperature (25°C) in order to perform the first ligation reaction (cf. Figure 19).

### Ligation protocol:

The ligation mixture contained oligonucleotides **I**, **II** and **III** (1.4fold excess of the phosphorylated oligonucleotide **II**) and 2x rapid ligation buffer (Biozym; 132 mM Tris-HCI, 20 mM MgCl₂, 2 mM DTT, 2 mM ATP, 15% PEG 6000, pH 7.6 @ 25°C). For the ligation reactions 600 units of T4 DNA ligase (600 U/µl; T4 DNA ligase rapid; Biozym) were added for a total reaction volume of 20 µl. The reactions mixtures were incubated overnight at 25°C and T4 DNA ligase was inactivated by incubating the samples at 75°C for 10 minutes.

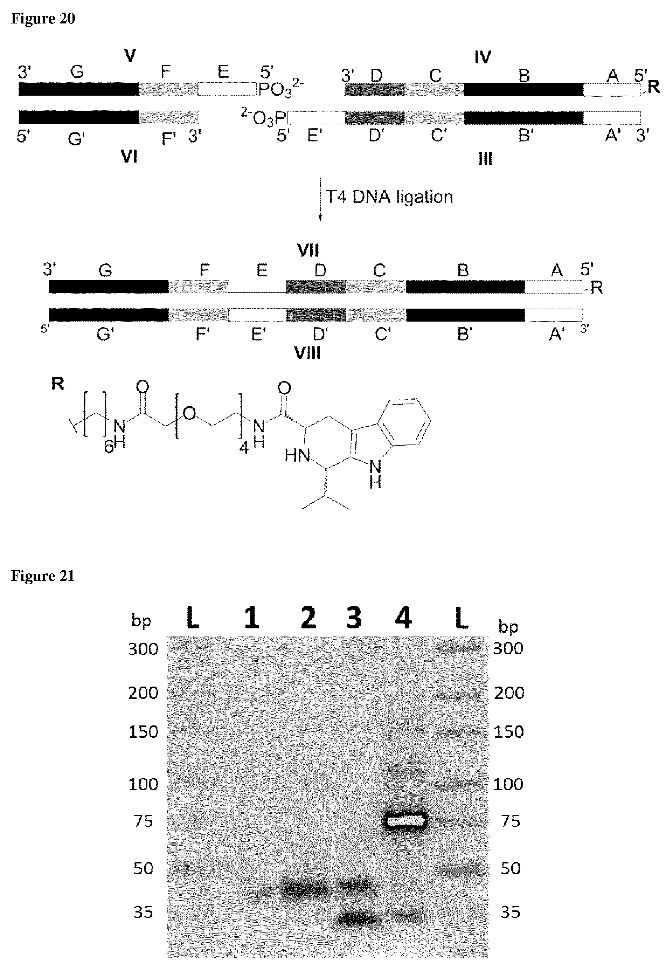

To confirm the ligation process, agarose gel electrophoresis was used to identify the components in the ligation reaction mixtures, shown in Figure 21. Electrophoresis was carried out in 1× TBE (pH 8.0) at 150 V constant voltage for 75 minutes. For staining, MIDORI DIRECT (Biozym) and as a reference ThermoScientific GeneRuler Ultra Low Range DNA Ladder were used. The second ligation reaction with T4 DNA ligase was prepared analogously. The scheme of the second ligation reaction is shown in Figure 20.

### MALDI MS analysis:

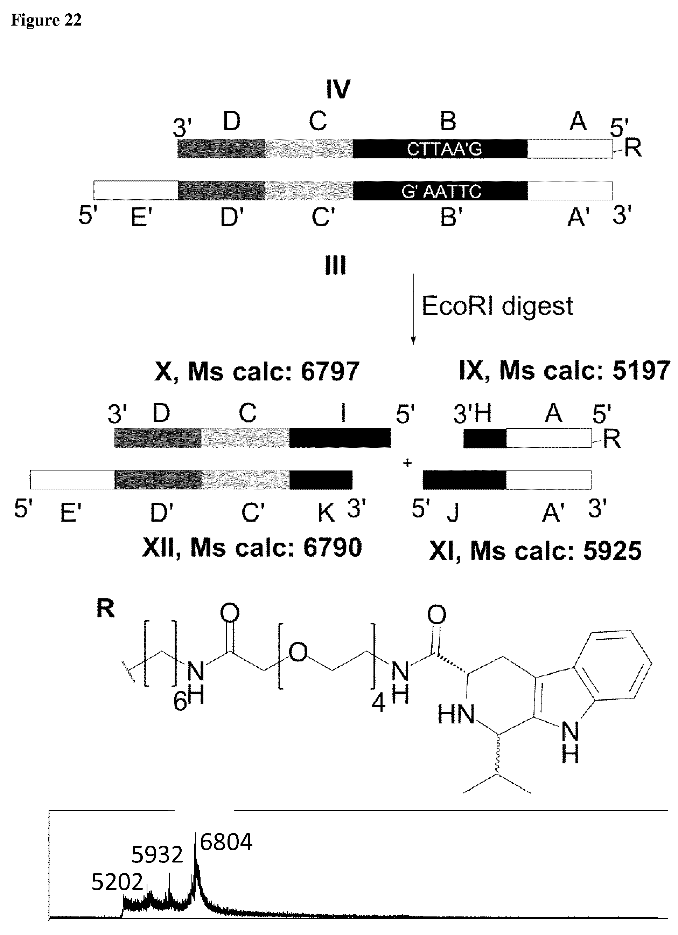

To confirm the ligation success for the first ligation reaction an EcoRI digest followed by MALDI-TOF analysis was performed (cf. Figure 22). For the EcoRI digest 25 pmol of the ligation product was incubated in 5 µl 10x Buffer H (commercially provided by Roche; 0,5 M Tris-HCl, 1 M NaCl, 100 mM MgCl₂, 10 mM DTE, pH 7,5 at 37°C) and 1 unit EcoRI. The total volume of the digest reaction was 50 µl (distilled water was added). The digest was performed at 37°C for overnight. Before measuring MALDI the reaction mixture was precipitated with 75% EtOH. For the MALDI analysis 3-HPA was used as matrix. The MALDI-TOF result shows 3 defined masses which correlate to the calculated masses (cf. Figure22).

In the following the DNA sequences, which involved in the ligation process, are depicted:

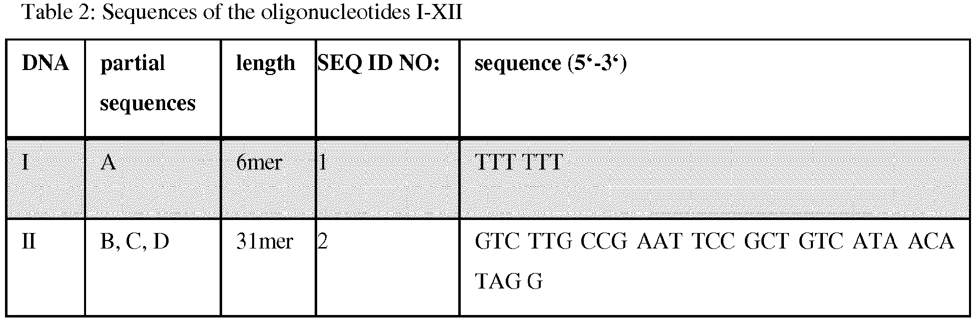

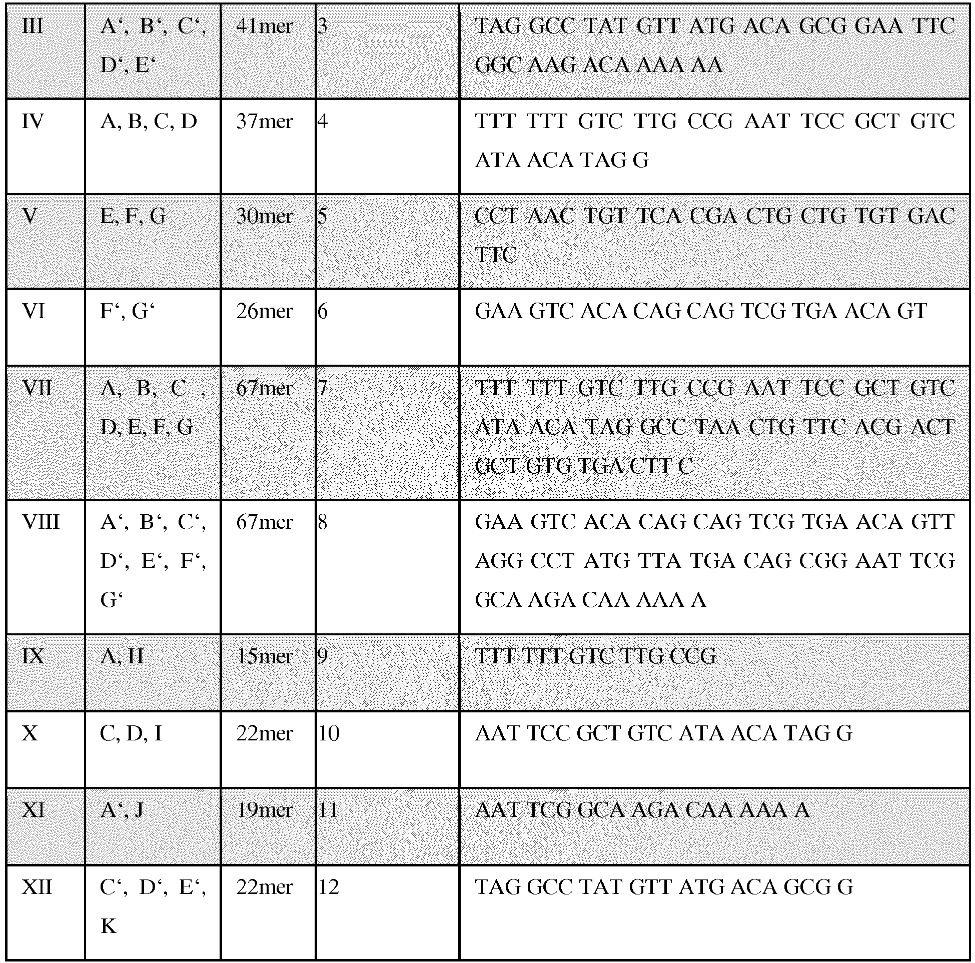

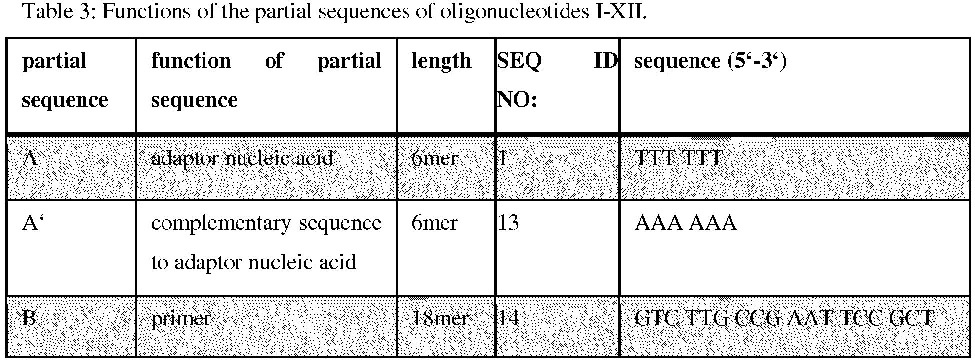

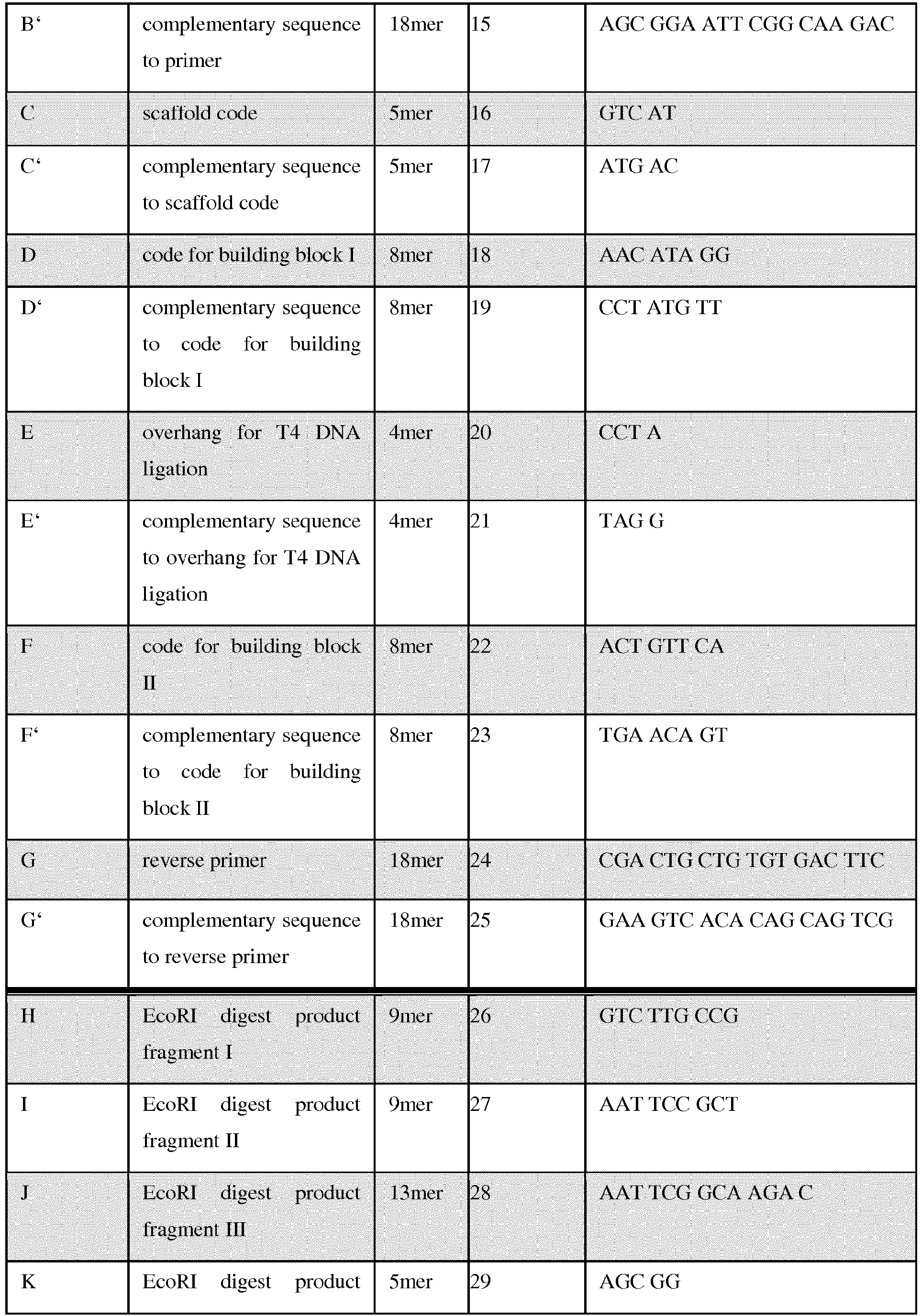

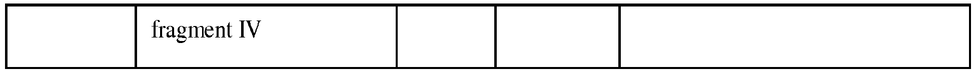

All documents cited herein, are hereby incorporated by reference in their entirety.

The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention. The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. In addition, where features or aspects of the invention are described in terms of Markush groups, those skilled in the art will recognize that the invention is also thereby described in terms of any individual member or subgroup of members of the Markush group. Further embodiments of the invention will become apparent from the following claims.

## Claims

1. Compound library comprising a plurality of conjugate molecules, wherein said conjugates comprise a small organic molecule covalently coupled to a nucleic acid moiety, wherein the nucleic acid moiety consists of pyrimidine nucleotides and the plurality of conjugates differ from each other at least by comprising different organic molecules.

2. The compound library according to claim 1, wherein the plurality of conjugate molecules comprises at least ten conjugate molecules, preferably at least 50 conjugate molecules, more preferably at least 100 conjugate molecules.

3. The compound library according to claim 1 or 2, wherein the nucleic acid moiety consists of two to 20, preferably three to ten, more preferably six nucleotides.

4. The compound library according to any one of claims 1 to 3, wherein the nucleic acid moiety is RNA, DNA or a mixture thereof.

5. The compound library according to claim 4, wherein the nucleic acid moiety is DNA.

6. The compound library according to any one of claims 1 to 5, wherein the nucleic acid moiety and the organic molecule are linked by amide bond.

7. The compound library according to any one of claims 1 to 6, wherein
a) the conjugates further comprise a linker between the nucleic acid moiety and the organic molecule, preferably this linker is polyethylene glycol (PEG);
b) the conjugates differ from each other by comprising different nucleic acid moieties;
c) the organic molecule consists of 2 or more carbon atoms, preferably up to 50 carbon atoms, more preferably up to 30 carbon atoms; and/or
d) the organic molecule has a molecular weight of at most 900 daltons, preferably at most 700 daltons and more preferably at most 500 daltons.

8. The compound library according to any one of claims 1 to 6, wherein the pyrimidine nucleotides are selected from the group consisting of thymine, cytosine, uracil, 4-acetylcytidine, 5-(carboxyhydroxymethyl)uridine, 2'-O-methylcytidine, 5-carboxymethylaminomethyl-2-thiouridine, 5-carboxymethylaminomethyluridine, dihydrouridine, 2'-O-methylpseudouridine, 1-methylpseudouridine, 3-methylcytidine, 5-methylcytidine, 5-methylaminomethyluridine, 5-methoxyaminomethyl-2-thiouridine, 5-methoxycarbonylmethyl-2-thiouridine, 5-methoxycarbonylmethyluridine, 5-methoxyuridine, uridine-5-oxyacetic acid-methylester, uridine-5-oxyacetic acid, pseudouridine, 2-thiocytidine, 5-methyl-2-thiouridine, 2-thiouridine, 4-thiouridine, 5-methyluridine, 2'-O-alkyluridine, 2'-O-alkylthymidine, 2'-O-alkylcytidine and 3-(3-amino-3-carboxypropyl)uridine.

9. Method for synthesizing a compound library, wherein said library comprises a plurality of conjugate molecules, wherein said conjugates comprise a small organic molecule covalently coupled to a nucleic acid moiety, the synthesis of each conjugate molecule comprises:
- reacting a nucleic acid consisting of pyrimidine nucleotides with an organic molecule under conditions to allow the conjugation of said molecules,
wherein the plurality of conjugates differ from each other at least by comprising different organic molecules.

10. The method according to claim 9, wherein the conjugation step of claim 9 comprises:
a) reacting the nucleic acid consisting of pyrimidine nucleotides with a first organic intermediate molecule under conditions to form a conjugate of the nucleic acid and the first organic intermediate molecule; and
b) reacting the conjugate of the nucleic acid and the first organic intermediate molecule with a second organic intermediate molecule to form the conjugate molecule.

11. The method according to claim 9 or 10, wherein the conjugate molecule and/or the conjugate of the nucleic acid and the first organic intermediate molecule is further used in a reaction comprising
a) a strong acid, preferably an acid having a pKₐ of less than 3; and/or
b) a catalyst selected from the group consisting of copper, silver, gold, ruthenium, iron, osmium, cobalt, rhodium, iridium, nickel, palladium, platinum and derivatives thereof, preferably copper, silver, gold, ruthenium and derivatives thereof.

12. The method according to any one of claims 9 to 11, wherein the nucleic acid moiety
a) consists of two to 20, preferably three to ten, more preferably six nucleotides;
b) is RNA, DNA or a mixture thereof, preferably DNA; and/or
c) is elongated in an additional reaction step with pyrimidine and/or purine nucleotides after the conjugation step.

13. The method according to any one of claims 9 to 12, wherein the nucleic acid moiety and the organic molecule are linked by amide bond.

14. The method according to any one of claims 9 to 13, wherein
a) the conjugates further comprise a linker between the nucleic acid moiety and the organic molecule, preferably this linker portion is polyethylene glycol (PEG);
b) the conjugates differ from each other by comprising different nucleic acid moieties;
c) the organic molecule consists of 2 or more carbon atoms, preferably up to 50 carbon atoms, more preferably up to 30 carbon atoms;
d) the organic molecule has a molecular weight of at most 900 daltons, preferably at most 700 daltons and more preferably at most 500 daltons; and/or
e) the pyrimidine nucleotides are selected from the group consisting of thymine, cytosine, uracil, 4-acetylcytidine, 5-(carboxyhydroxymethyl)uridine, 2'-O-methylcytidine, 5-carboxymethylaminomethyl-2-thiouridine, 5-carboxymethylaminomethyluridine, dihydrouridine, 2'-O-methylpseudouridine, 1-methylpseudouridine, 3-methylcytidine, 5-methylcytidine, 5-methylaminomethyluridine, 5-methoxyaminomethyl-2-thiouridine, 5-methoxycarbonylmethyl-2-thiouridine, 5-methoxycarbonylmethyluridine, 5-methoxyuridine, uridine-5-oxyacetic acid-methylester, uridine-5-oxyacetic acid, pseudouridine, 2-thiocytidine, 5-methyl-2-thiouridine, 2-thiouridine, 4-thiouridine, 5-methyluridine, 2'-O-alkyluridine, 2'-O-alkylthymidine, 2'-O-alkylcytidine and 3-(3-amino-3-carboxy-propyl)uridine.

15. Use of a compound library according to any one of claims 1 to 8 for screening a compound binding to a target molecule, preferably the target molecule is a protein.
